(19) Europäisches Patentamt
European Patent Office
Office européen des brevets

(11) **EP 3 081 216 A1**

(12) **EUROPEAN PATENT APPLICATION**

(43) Date of publication:
**19.10.2016 Bulletin 2016/42**

(51) Int Cl.:
**A61K 31/496** (2006.01)   **C07D 215/22** (2006.01)
**A61P 25/18** (2006.01)

(21) Application number: **16169141.5**

(22) Date of filing: **25.09.2002**

(84) Designated Contracting States:
**AT BE BG CH CY CZ DE DK EE ES FI FR GB GR IE IT LI LU MC NL PT SE SK TR**
Designated Extension States:
**LT LV RO SI**

(30) Priority: **25.09.2001   JP 2001290645**
**14.11.2001   JP 2001348276**
**27.03.2002   CA 2379005**

(62) Document number(s) of the earlier application(s) in accordance with Art. 76 EPC:
**08000358.5 / 1 925 308**
**04002427.5 / 1 419 776**
**02782507.4 / 1 330 249**

(71) Applicant: **OTSUKA PHARMACEUTICAL CO., LTD.**
**Chiyoda-ku**
**Tokyo 101 (JP)**

(72) Inventors:
• **Bando, Takuji**
  **Tokushima, 770-0045 (JP)**
• **Aoki, Satoshi**
  **Tokushima, 772-0001 (JP)**
• **Kawasaki, Junichi**
  **Tokushima, 771-0204 (JP)**
• **Ishigami, Makoto**
  **Tokushima, 771-1273 (JP)**
• **Taniguchi, Youichi**
  **Tokushima, 770-0861 (JP)**
• **Yabuuchi, Tsuyoshi**
  **Tokushima, 771-0131 (JP)**
• **Fujimoto, Kiyoshi**
  **Tokushima, 772-0035 (JP)**

• **Nishioka, Yoshihiro**
  **Tokushima, 771-0212 (JP)**
• **Kobayashi, Noriyuki**
  **Tokushima, 770-0944 (JP)**
• **Fujimura, Tsutomu**
  **Tokushima, 772-0035 (JP)**
• **Takahashi, Masanori**
  **Tokushima, 770-8025 (JP)**
• **Abe, Kaoru**
  **Tokushima, 770-0866 (JP)**
• **Nakagawa, Tomonori**
  **Tokushima, 771-0219 (JP)**
• **Shinhama, Koichi**
  **Tokushima, 770-0812 (JP)**
• **Utsumi, Naoto**
  **Tokushima, 772-0014 (JP)**
• **Tominaga, Michiaki**
  **Tokushima, 771-1346 (JP)**
• **Oi, Yoshihiro**
  **Tokushima, 770-0003 (JP)**
• **Yamada, Shohei**
  **Tokushima, 771-1231 (JP)**
• **Tomikawa, Kenji**
  **Tokushima, 779-3118 (JP)**

(74) Representative: **Hoffmann Eitle**
**Patent- und Rechtsanwälte PartmbB**
**Arabellastraße 30**
**81925 München (DE)**

Remarks:
This application was filed on 11.05.2016 as a divisional application to the application mentioned under INID code 62.

(54) **PHARMACEUTICAL SOLID ORAL ARIPIPRAZOLE PREPARATION AND PROCESSES FOR THE PREPARATION THEREOF**

(57)    A pharmaceutical solid oral aripiprazole preparation is provided which has
a) at least one dissolution rate measured after storage in the open at 25°C/60% RH for six months selected from the group consisting of 70 % or more at pH 4.5 after 60 minutes, and 55 % or more at pH 5.0 after 60 minutes; or
b) at least one dissolution rate measured after storage in the open at 40°C/75% RH for 1 week selected from the group consisting of 70 % or more at pH 4.5 after 60 minutes, and 55 % or more at pH 5.0 after 60 minutes.

EP 3 081 216 A1

**Description**

DETAILED DESCRIPTION OF THE INVENTION

Field of the Invention

The present invention relates to an improved form of aripiprazole having reduced hygroscopicity and processes for the preparation of this improved form.

Background of the Invention

Aripiprazole, 7-(4-[4-(2,3-dichlorophenyl)-1-piperazinyl]-butoxy}-3,4-dihydro carbostyril or 7-{4-[4-(2,3-dichlorophenyl)-1-piperazinyl]-butoxy)-3,4-dihydro-2(1H)-quinolinone, is an atypical antipsychotic agent useful for the treatment of schizophrenia (U.S. 4,734,416 and U.S. 5,006,528). Schizophrenia is a common type of psychosis characterized by delusions, hallucinations and extensive withdrawal from others. Onset of schizophrenia typically occurs between the age of 16 and 25 and affects 1 in 100 individuals worldwide. It is more prevalent than Alzheimer's disease, multiple sclerosis, insulin-dependent diabetes and muscular dystrophy. Early diagnosis and treatment can lead to significantly improved recovery and outcome. Moreover, early therapeutic intervention can avert costly hospitalization.

According to Example 1 of Japanese Unexamined Patent Publication No. 191256/1990, aripiprazole anhydride crystals are manufactured for example by reacting 7-(4-bromobutoxy)-3,4-dihydrocarbostyril with 1-(2,3-dichlorophenylpiperadine and recrystallizing the resulting raw aripiprazole anhydride with ethanol. Also, according to the Proceedings of the 4th Japanese-Korean Symposium on Separation Technology (October 6-8, 1996), aripiprazole anhydride crystals are manufactured by heating aripiprazole hydrate at 80°C. However, the aripiprazole anhydride crystals obtained by the aforementioned methods have the disadvantage of being significantly hygroscopic.

The hygroscopicity of these crystals makes them difficult to handle since costly and burdensome measures must be taken in order ensure they are not exposed to moisture during process and formulation. Exposed to moisture, the anhydrous form can take on water and convert to a hydrous form. This presents several disadvantages. First, the hydrous forms of aripiprazole have the disadvantage of being less bioavailable and less dissoluble than the anhydrous forms of aripiprazole. Second, the variation in the amount of hydrous versus anhydrous aripiprazole drug substance from batch to batch could fail to meet specifications set by drug regulatory agencies. Third, the milling may cause the drug substance, Conventional Anhydride, to adhere to manufacturing equipment which may further result in processing delay, increased operator involvement, increased cost, increased maintenance and lower production yield. Fourth, in addition to problems caused by introduction of moisture during the processing of these hygroscopic anhydrides, the potential for absorbance of moisture during storage and handling would adversely affect the dissolubility of aripiprazole drug substance. Thus shelf-life of the product could be significantly decreased and/or packaging costs could be significantly increased. It would be highly desirable to discover a form of aripiprazole that possessed low hygroscopicity thereby facilitating pharmaceutical processing and formulation operations required for producing dosage units of an aripiprazole medicinal product having improved shelf-life, suitable dissolubility and suitable bioavailability.

Also, Proceedings of the 4th Japanese-Korean Symposium on Separation Technology (October 6-8, 1996) state that, aripiprazole anhydride crystals exist as type-I crystals and type-II crystals; the type-I crystals of aripiprazole anhydride can be prepared by recrystallizing from an ethanol solution of aripiprazole, or by heating aripiprazole hydrate at 80°C; and the type-II crystals of aripiprazole anhydride can be prepared by heating the type-I crystals of aripiprazole anhydride at 130 to 140°C for 15 hours.

By the aforementioned methods, aripiprazole anhydride type-II crystals having high purity can not be easily prepared in an industrial scale with good repeatability.

SUMMARY OF THE INVENTION

Thus according to the present invention is provided a form of aripiprazole having reduced hygroscopicity and which is more amenable to pharmaceutical processing and formulation. The inventors of the present invention have discovered that this reduced-hygroscopic form of Aripiprazole is a crystalline substance defined herein as Anhydride B. A particular process for the preparation of this anhydrous crystalline substance has also been discovered and comprises yet another aspect of the present invention. Particularly, it was discovered as part of the present invention that in order to produce Anhydride B having the desired pharmaceutical properties and utilizing the most efficient process, Hydrate A, as defined herein, would have to serve as the intermediate. It was also discovered that a particular sequence of processing had to be implemented in order to form Hydrate A. It was discovered that the preparation of Hydrate A required milling what is defined herein as Conventional Hydrate. Then, Hydrate A can be transformed into Anhydride B through suitable heating as defined herein. Surprisingly, if the Conventional Hydrate is first heated and then milled, serious agglomeration sets in rendering the processing commercially unsuitable.

An object of the present invention is to provide novel aripiprazole anhydride crystals.

Moreover, another object of the present invention is to provide aripiprazole anhydride crystals which neither easily convert into hydrates nor substantially decrease the original solubility, even when a pharmaceutical composition comprising aripiprazole anhydride is stored for a long period of time.

Further object of the present invention is to provide preparation methods, in order to obtain aripiprazole anhydride crystals having high purity in an industrial scale with good repeatability.

The present inventors have conducted research works aimed to attain the aforementioned objects. In the course of the research, they have found that the desired aripiprazole anhydride crystals can be obtained when a well-known aripiprazole anhyride is heated at the specific temperature. Further, the present inventors have found that the desired aripiprazole anhydride crystals can be obtained from recrystallization of a well-known aripiprazole anhydride by using the specific solvents. Moreover, the present inventors found that the desired aripiprazole anhydride crystals can be obtained by suspending a well-known aripiprazole anhydride in the specific solvent, and heating thus obtained suspension.

The present invention thus completed on the basis of these findings and knowledge.

BRIEF DESCRIPTION OF THE DRAWINGS

Figure 1 is a thermogravimetric/differential thermogram of the Aripiprazole Hydrate A obtained in Example 1.

Figure 2 shows the $^1$H-NMR spectrum (DMSO-d$_6$, TMS) of the Aripiprazole Hydrate A obtained in Example 1.

Figure 3 is a powder x-ray diffraction diagram of the Aripiprazole Hydrate A obtained in Example 1.

Figure 4 shows the $^1$H-NMR spectrum (DMSO-d$_6$, TMS) of the Aripiprazole Anhydride Crystals B obtained in Example 2.

Figure 5 is a powder x-ray diffraction diagram of the Aripiprazole Anhydride Crystals B obtained in Example 2.

Figure 6 is a thermogravimetric/differential thermogram of the aripiprazole hydrate obtained in Reference Example 3.

Figure 7 is a powder x-ray diffraction diagram of the aripiprazole hydrate obtained in Reference Example 3.

Figure 8 shows thermogravimetric/differential thermal analysis endothermic curve of the type C crystals of aripiprazole anhydride obtained in Example 11.

Figure 9 shows an $^1$H-NMR spectrum (DMSO-d$_6$, TMS) of the type C crystals of aripiprazole anhydride obtained in Example 11.

Figure 10 shows a powder X-ray diffraction spectrum of the type C crystals of aripiprazole anhydride obtained in Example 11.

Figure 11 shows an IR spectrum of the type C crystals of aripiprazole anhydride obtained in Example 11.

Figure 12 shows a solid $^{13}$C-NMR spectrum of the type C crystals of aripiprazole anhydride obtained in Example 11.

Figure 13 shows a thermogravimetric/ differential thermal analysis endothermic curve of the type D crystals of aripiprazole anhydride obtained in Example 12 or Example 13.

Figure 14 shows an $^1$H-NMR spectrum (DMSO-d$_6$, TMS) of the type D crystals of aripiprazole anhydride obtained in Example 12 or Example 13.

Figure 15 shows a powder X-ray diffraction spectrum of the type D crystals of aripiprazole anhydride obtained in Example 12 or Example 13.

Figure 16 shows an IR spectrum of the type D crystals of aripiprazole anhydride obtained in Example 12 or Example 13.

Figure 17 shows a solid $^{13}$C-NMR spectrum of the type D crystals of aripiprazole anhydride obtained in Example 12 or Example 13.

Figure 18 shows a thermogravimetric/ differential thermal analysis endothermic curve of the type E crystals of aripiprazole anhydride obtained in Example 14.

Figure 19 shows an $^1$H-NMR spectrum (DMSO-d$_6$, TMS) of the type E crystals of aripiprazole anhydride obtained in Example 14.

Figure 20 shows a powder X-ray diffraction spectrum of the type E crystals of aripiprazole anhydride obtained in Example 14.

Figure 21 shows an IR spectrum of the type E crystals of aripiprazole anhydride obtained in Example 14.

Figure 22 shows a thermogravimetric/ differential thermal analysis endothermic curve of the type F crystals of aripiprazole anhydride obtained in Example 15.

Figure 23 shows an $^1$H-NMR spectrum (DMSO-d$_6$, TMS) of the type F crystals of aripiprazole anhydride obtained in Example 15.

Figure 24 shows a powder X-ray diffraction spectrum of the type F crystals of aripiprazole anhydride obtained in Example 15.

Figure 25 shows an IR spectrum of the type F crystals of aripiprazole anhydride obtained in Example 15.

Figure 26 shows thermogravimetric/ differential thermal analysis endothermic curve of the type G crystals of aripiprazole anhydride obtained in Example 16-b).

Figure 27 shows an $^1$H-NMR spectrum (DMSO-$d_6$, TMS) of the type G crystals of aripiprazole anhydride obtained in Example 16-b).

Figure 28 shows a powder X-ray diffraction spectrum of the type G crystals of aripiprazole anhydride obtained in Example 16-b).

Figure 29 shows an IR spectrum of the type G crystals of aripiprazole anhydride obtained in Example 16-b).

Figure 30 shows a thermogravimetric/ differential thermal analysis endothermic curve of the glass form of aripiprazole anhydride obtained in Example 16-a).

Figure 31 shows a powder X-ray diffraction spectrum of the glassy state of aripiprazole anhydride obtained in Example 16-a).

## DETAILED DESCRIPTION OF THE INVENTION

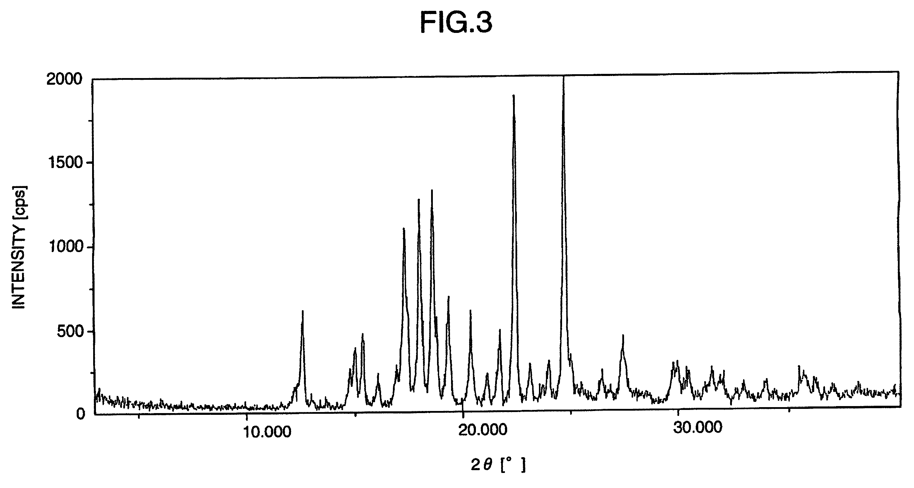

According to first embodiment of the first aspect of the present invention is provided Hydrate A of aripiprazole wherein said Hydrate has a powder x-ray diffraction spectrum which is substantially the same as the powder x-ray diffraction spectrum shown in Figure 3.

According to another embodiment of the first aspect of the present invention is provided Hydrate A of aripiprazole wherein said Hydrate has powder x-ray diffraction characteristic peaks at $2\theta$ = 12.6°, 15.4°, 17.3°, 18.0°, 18.6°, 22.5° and 24.8°.

According to another embodiment of the first aspect of the present invention is provided Hydrate A of aripiprazole wherein said Hydrate has particular infrared absorption bands at 2951, 2822, 1692, 1577, 1447, 1378, 1187, 963 and 784 cm$^{-1}$ on the IR (KBr) spectrum.

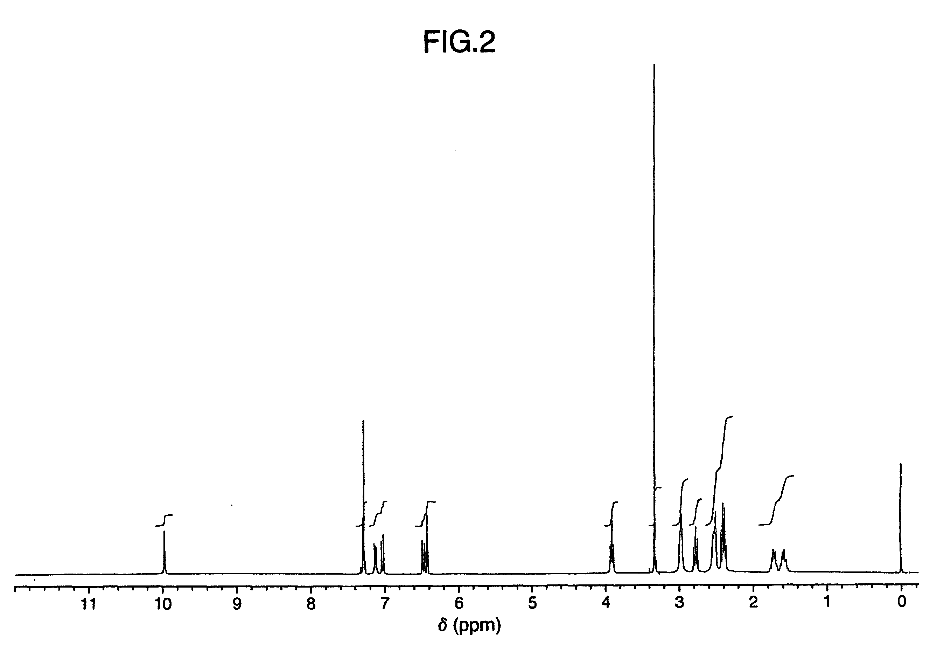

According to another embodiment of the first aspect of the present invention is provided Hydrate A of aripiprazole wherein said Hydrate has an $^1$H-NMR spectrum which is substantially the same as the $^1$H-NMR spectrum (DMSO-$d_6$, TMS) shown in Figure 2.

According to another embodiment of the first aspect of the present invention is provided Hydrate A of aripiprazole wherein said Hydrate has an $^1$H-NMR spectrum (DMSO-$d_6$, TMS) having characteristic peaks at 1.55-1.63 ppm (m, 2H), 1.68-1.78 ppm (m, 2H), 2.35-2.46 ppm (m, 4H), 2.48-2.56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7.17 ppm (m, 1H), 7.28-7.32 ppm (m, 2H) and 10.00 ppm (s, 1H).

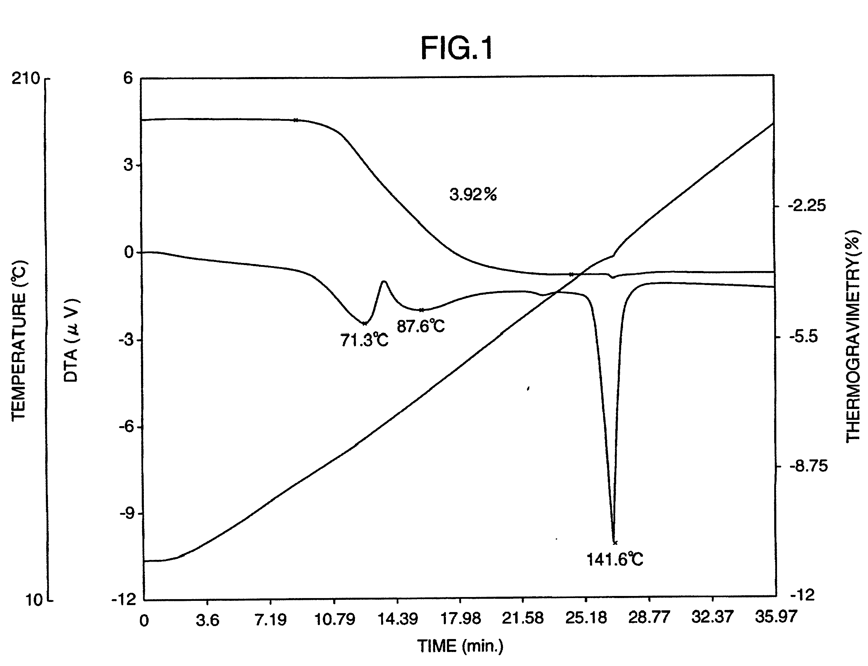

According to another embodiment of the first aspect of the present invention is provided Hydrate A of aripiprazole wherein said Hydrate has an endothermic curve which is substantially the same as the thermogravimetric/differential thermal analysis (heating rate 5°C/min) endothermic curve shown in Figure 1.

According to another embodiment of the first aspect of the present invention is provided Hydrate A of aripiprazole wherein said Hydrate has a mean particle size of 50 $\mu$m or less.

According to another embodiment of the first aspect of the present invention is provided Hydrate A of aripiprazole wherein said Hydrate has a mean particle size of 40 $\mu$m or less.

According to another embodiment of the first aspect of the present invention is provided Hydrate A of aripiprazole wherein said Hydrate has a mean particle size of 35 $\mu$m or less.

According to another embodiment of the first aspect of the present invention is provided Hydrate A of aripiprazole wherein said Hydrate has a mean particle size of 30 $\mu$m or less.

According to another embodiment of the first aspect of the present invention is provided Hydrate A of aripiprazole wherein said Hydrate has a mean particle size of 25 $\mu$m or less.

According to another embodiment of the first aspect of the present invention is provided Hydrate A of aripiprazole wherein said Hydrate has a mean particle size of 20 $\mu$m or less.

According to another embodiment of the first aspect of the present invention is provided Hydrate A of aripiprazole wherein said Hydrate has a mean particle size range of 40 to 10 $\mu$m.

According to another embodiment of the first aspect of the present invention is provided Hydrate A of aripiprazole wherein said Hydrate has a mean particle size range of 36 to 14 $\mu$m.

According to a second aspect of the present invention is provided a process for the preparation of Hydrate A wherein said process comprises the steps of milling Conventional Hydrate.

According to a first embodiment of the second aspect of the present invention is provided a process for the preparation of Hydrate A comprising the steps of milling Conventional Hydrate wherein said milling is performed by a milling machine.

According to another embodiment of the second aspect of the present invention is provided a process for the preparation of Hydrate A comprising the steps of milling Conventional Hydrate wherein said milling machine is an atomizer, pin mill, jet mill or ball mill.

According to another embodiment of the second aspect of the present invention is provided a process for the preparation of Hydrate A comprising the steps of milling Conventional Hydrate wherein said milling machine is an atomizer.

According to another embodiment of the second aspect of the present invention is provided a process for the preparation of Hydrate A comprising the steps of milling Conventional Hydrate wherein said milling machine is an atomizer using a rotational speed of 5000-15000 rpm for the main axis, a feed rotation of 10-30 rpm and a screen hole size of 1-5 mm.

According to various embodiments of a third aspect of the present invention is provided Hydrate A defined according to one or more of the embodiments described herein wherein said Hydrate is made by a process as described herein.

According to a fourth aspect of the present invention is provided aripiprazole drug substance of low hygroscopicity.

According to a first embodiment of the fourth aspect of the present invention is provided aripiprazole drug substance of low hygroscopicity wherein said low hygroscopicity is a moisture content of 0.5% or less after placing said drug substance for 24 hours in a dessicator maintained at a temperature of 60°C and a humidity level of 100%.

According to a first embodiment of the fourth aspect of the present invention is provided aripiprazole drug substance of low hygroscopicity wherein said low hygroscopicity is a moisture content of 0.4% or less after placing said drug substance for 24 hours in a dessicator maintained at a temperature of 60°C and a humidity level of 100%.

According to another embodiment of the fourth aspect of the present invention is provided aripiprazole drug substance of low hygroscopicity wherein said low hygroscopicity is a moisture content of 0.25% or less after placing said drug substance for 24 hours in a dessicator maintained at a temperature of 60°C and a humidity level of 100%.

According to another embodiment of the fourth aspect of the present invention is provided aripiprazole drug substance of low hygroscopicity wherein said low hygroscopicity is a moisture content of 0.15% or less after placing said drug substance for 24 hours in a dessicator maintained at a temperature of 60°C and a humidity level of 100%.

According to another embodiment of the fourth aspect of the present invention is provided aripiprazole drug substance of low hygroscopicity wherein said low hygroscopicity is a moisture content of 0.10% or less after placing said drug substance for 24 hours in a dessicator maintained at a temperature of 60°C and a humidity level of 100%.

According to another embodiment of the fourth aspect of the present invention is provided aripiprazole drug substance of low hygroscopicity wherein said low hygroscopicity is a moisture content of 0.05% or less after placing said drug substance for 24 hours in a dessicator maintained at a temperature of 60°C and a humidity level of 100%.

According to another embodiment of the fourth aspect of the present invention is provided aripiprazole drug substance of low hygroscopicity wherein said low hygroscopicity is a moisture content of 0.04% or less after placing said drug substance for 24 hours in a dessicator maintained at a temperature of 60°C and a humidity level of 100%.

According to another embodiment of the fourth aspect of the present invention is provided aripiprazole drug substance of low hygroscopicity wherein said drug substance is Aripiprazole Anhydride Crystals B as defined herein.

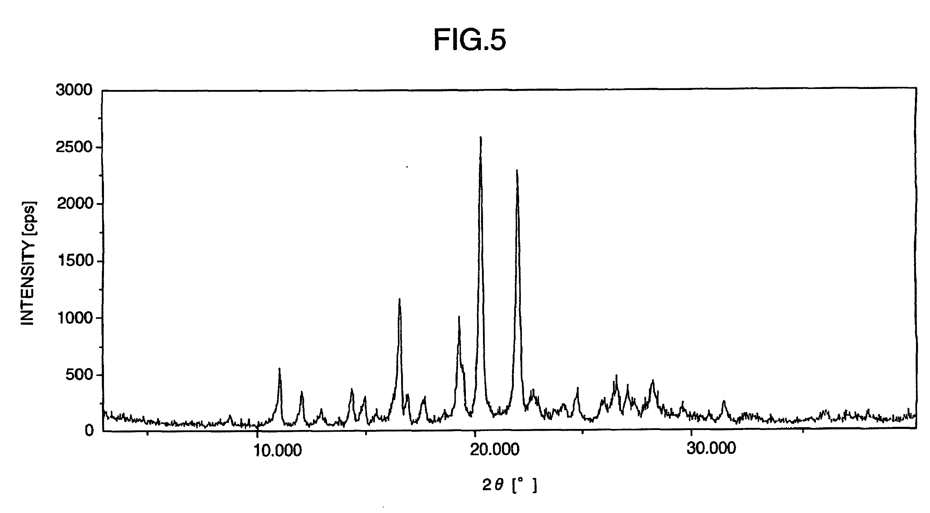

According to another embodiment of the fourth aspect of the present invention is provided aripiprazole drug substance of low hygroscopicity wherein said drug substance has a powder x-ray diffraction spectrum which is substantially the same as the powder x-ray diffraction spectrum shown in Figure 5.

According to another embodiment of the fourth aspect of the present invention is provided aripiprazole drug substance of low hygroscopicity wherein said drug substance has a powder x-ray diffraction spectrum having characteristic peaks at $2\theta$ = 11.0°, 16.6°, 19.3°, 20.3° and 22.1°.

According to another embodiment of the fourth aspect of the present invention is provided aripiprazole drug substance of low hygroscopicity wherein said drug substance has particular infrared absorption bands at 2945, 2812, 1678, 1627, 1448, 1377, 1173, 960 and 779 $cm^{-1}$ on the IR (KBr) spectrum.

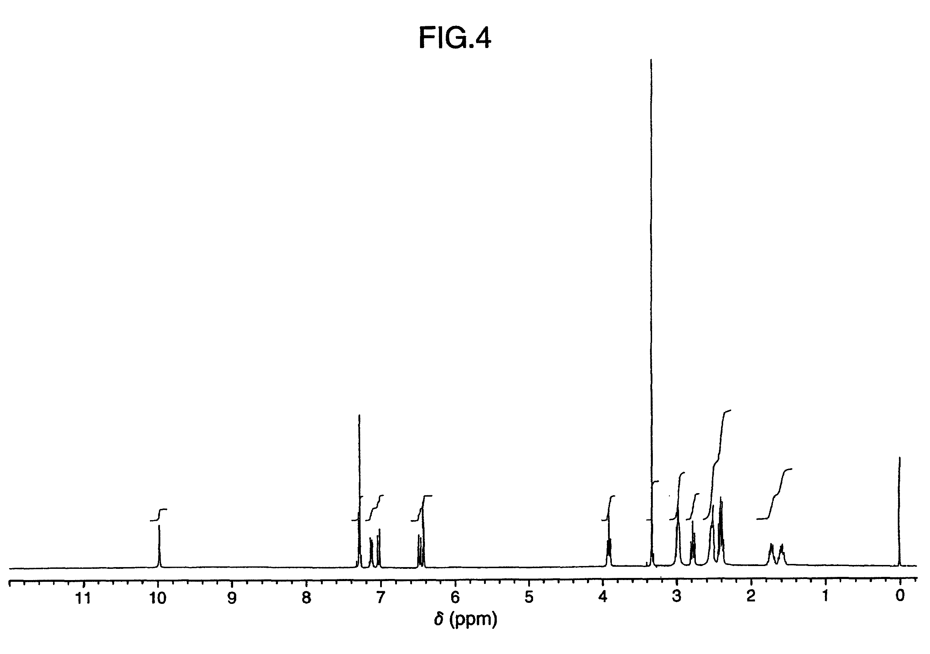

According to another embodiment of the fourth aspect of the present invention is provided aripiprazole drug substance of low hygroscopicity wherein said drug substance has an $^1$H-NMR spectrum which is substantially the same as the $^1$H-NMR spectrum (DMSO-$d_6$, TMS) shown in Figure 4.

According to another embodiment of the fourth aspect of the present invention is provided aripiprazole drug substance of low hygroscopicity wherein said drug substance has an $^1$H-NMR spectrum (DMSO-$d_6$, TMS) having characteristic peaks at 1.55-1.63 ppm (m, 2H), 1.68-1.78 ppm (m, 2H), 2.35-2.46 ppm (m, 4H), 2.48-2.56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7.17 ppm (m, 1H), 7.28-7.32 ppm (m, 2H) and 10.00 ppm (s, 1H).

According to another embodiment of the fourth aspect of the present invention is provided aripiprazole drug substance of low hygroscopicity wherein said drug substance exhibits an endothermic peak near about 141.5°C in thermogravimetric/ differential thermal analysis (heating rate 5°C/min).

According to another embodiment of the fourth aspect of the present invention is provided aripiprazole drug substance of low hygroscopicity wherein said drug substance exhibits an endothermic peak near about 140.7°C in differential scanning calorimetry (heating rate 5°C/min).

According to another embodiment of the fourth aspect of the present invention is provided aripiprazole drug substance of low hygroscopicity wherein said drug substance is Aripiprazole Anhydride Crystals B and will not substantially convert to a hydrous form of aripiprazole when properly stored even for an extended period. For instance, said Aripiprazole Anhydride Crystals B can be stored under a relative humidity (RH) of 60 % and at a temperature of 25°C, even for a period not less than 1 year.

According to another embodiment of the fourth aspect of the present invention is provided aripiprazole drug substance of low hygroscopicity wherein said drug substance is Aripiprazole Anhydride Crystals B and will not substantially convert to a hydrous form of aripiprazole when properly stored even for an extended period. For instance, said Aripiprazole Anhydride Crystals B can be stored under a relative humidity (RH) of 60 % and at a temperature of 25°C, even for a period not less than 4 years.

According to another embodiment of the fourth aspect of the present invention is provided aripiprazole drug substance of low hygroscopicity wherein said drug substance is Aripiprazole Anhydride Crystals B and will not substantially convert to a hydrous form of aripiprazole when properly stored even for a period not less than 0.5 year under a relative humidity (RH) of 75 % and at a temperature of 40°C.

According to another embodiment of the fourth aspect of the present invention is provided aripiprazole drug substance of low hygroscopicity wherein said drug substance has a mean size of 50μm or less when small particle size is required for the formulation such as Tablet and other solid dose formulations including for example flashmelt formulations.

According to another embodiment of the fourth aspect of the present invention is provided aripiprazole drug substance of low hygroscopicity wherein said drug substance has a mean size of 40μm or less if small particle size is required for the formulation such as Tablet and other solid dose formulations including for example flashmelt formulations.

According to another embodiment of the fourth aspect of the present invention is provided aripiprazole drug substance of low hygroscopicity wherein said drug substance has a mean size of 30μm or less if small particle size is required for formulation such as Tablet and other solid dose formulations including for example flashmelt formulations.

According to a fifth aspect of the present invention is provided a process for the preparation of Aripiprazole Anhydride Crystals B.

According to a first embodiment of the fifth aspect of the present invention is provided a process for the preparation of Aripiprazole Anhydride Crystals B wherein said process comprises heating Aripiprazole Hydrate A.

According to a first embodiment of the fifth aspect of the present invention is provided a process for the preparation of Aripiprazole Anhydride Crystals B wherein said process comprises heating Aripiprazole Hydrate A at 90-125°C for about 3-50 hours.

According to another embodiment of the fifth aspect of the present invention is provided a process for the preparation of Aripiprazole Anhydride Crystals B wherein said process comprises heating Aripiprazole Hydrate A at 100°C for about 18 hours.

According to another embodiment of the fifth aspect of the present invention is provided a process for the preparation of Aripiprazole Anhydride Crystals B wherein said process comprises heating Aripiprazole Hydrate A at 100°C for about 24 hours.

According to another embodiment of the fifth aspect of the present invention is provided a process for the preparation of Aripiprazole Anhydride Crystals B wherein said process comprises heating Aripiprazole Hydrate A at 120°C for about 3 hours.

According to another embodiment of the fifth aspect of the present invention is provided a process for the preparation of Aripiprazole Anhydride Crystals B wherein said process comprises heating Aripiprazole Hydrate A for about 18 hours at 100°C followed by additional heating for about 3 hours at 120°C.

According to a sixth aspect of the present invention is provided Aripiprazole Anhydride Crystals B defined according to one or more of the embodiments described herein and made by a process as provided herein.

According to a seventh aspect of the present invention is provided Aripiprazole Anhydride Crystals B formulated with one or more pharmaceutically acceptable carriers.

Other embodiments of the present invention may comprise suitable combinations of two or more of the embodiments and/or aspects disclosed herein.

Yet other embodiments and aspects of the invention will be apparent according to the description provided below.

Yet another aspect of the present invention comprised discovering that when aripiprazole hydrate (Conventional Hydrate as defined herein) is milled, it converts to an aripiprazole hydrate (Hydrate A as defined herein) with a different powder x-ray diffraction spectrum by different peak intensities. Moreover, it was found that Hydrate A loses the sharp dehydration endothermic peak of 123.5°C which characterizes unmilled Conventional Hydrate in thermogravimetric/differential thermal analysis. Thus, the Conventional Hydrate is transformed into Hydrate A after milling Conventional Hydrate and exhibits a gradual dehydration endothermic peak between about 60°C and 120°C with a weak peak at about 71°C.

Yet another aspect of the invention comprised discovering that when heated to a specific temperature of 90-125°C for 3-50hr, this novel aripiprazole hydrate dehydrates gradually avoiding the aggregation phenomenon thought to be caused in conventional aripiprazole hydrate by rapid dehydration, and that aripiprazole anhydride crystals obtained by heating of the novel aripiprazole hydrate to a specific temperature are aripiprazole anhydride crystals with the desired properties.

Characterization of Hydrate A

Particles of "Hydrate A" as used herein have the physicochemical properties given in (1)-(5) below:

(1) It has an endothermic curve which is substantially the same as the thermogravimetric/ differential thermal analysis (heating rate 5°C/min) endothermic curve shown in Figure 1. Specifically, it is characterized by the appearance of a small peak at about 71°C and a gradual endothermic peak around 60°C to 120°C.
(2) It has an $^1$H-NMR spectrum which is substantially the same as the $^1$H-NMR spectrum (DMSO-$d_6$, TMS) shown in Figure 2. Specifically, it has characteristic peaks at 1.55-1.63 ppm (m, 2H), 1.68-1.78 ppm (m, 2H), 2.35-2.46 ppm (m, 4H), 2.48-2.56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7.17 ppm (m, 1H), 7.28-7.32 ppm (m, 2H) and 10.00 ppm (s, 1H).
(3) It has a powder x-ray diffraction spectrum which is substantially the same as the powder x-ray diffraction spectrum shown in Figure 3. Specifically, it has characteristic peaks at $2\theta$ = 12.6°, 15.4°, 17.3°, 18.0°, 18.6°, 22.5° and 24.8°.
(4) It has clear infrared absorption bands at 2951, 2822, 1692, 1577, 1447, 1378, 1187, 963 and 784 cm$^{-1}$ on the IR (KBr) spectrum.
(5) It has a mean particle size of 50 $\mu$m or less.

Process for Manufacturing Hydrate A

Hydrate A is manufactured by milling Conventional Hydrate. Conventional milling methods can be used to mill Conventional Hydrate. For example, Conventional Hydrate can be milled in a milling machine. A widely used milling machine can be used, such as an atomizer, pin mill, jet mill or ball mill. Of these, the atomizer is preferred.
Regarding the specific milling conditions when using an atomizer, a rotational speed of 5000-15000 rpm could be used for the main axis, for example, with a feed rotation of 10-30 rpm and a screen hole size of 1-5 mm.
The mean particle size of the Aripiprazole Hydrate A obtained by milling should normally be 50 $\mu$m or less, preferably 30 $\mu$m or less. Mean particle size can be ascertained by the particle size measurement method described hereinafter.

Characterization of Aripiprazole Anhydride Crystals B

"Aripiprazole Anhydride Crystals B" of the present invention as used herein have the physicochemical properties given in (6)-(12) below.

(6) They have an $^1$H-NMR spectrum which is substantially the same as the $^1$H-NMR spectrum (DMSO-$d_6$, TMS) shown in Figure 4. Specifically, they have characteristic peaks at 1.55-1.63 ppm (m, 2H), 1.68-1.78 ppm (m, 2H), 2.35-2.46 ppm (m, 4H), 2.48-2.56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7.17 ppm (m, 1H), 7.28-7.32 ppm (m, 2H) and 10.00 ppm (s, 1H).
(7) They have a powder x-ray diffraction spectrum which is substantially the same as the powder x-ray diffraction spectrum shown in Figure 5. Specifically, they have characteristic peaks at $2\theta$ = 11.0°, 16.6°, 19.3°, 20.3° and 22.1°.
(8) They have clear infrared absorption bands at 2945, 2812, 1678, 1627, 1448, 1377, 1173, 960 and 779 cm$^{-1}$ on the IR (KBr) spectrum.
(9) They exhibit an endothermic peak near about 141.5°C in thermogravimetric/differential thermal analysis (heating rate 5°C/min).
(10) They exhibit an endothermic peak near about 140.7°C in differential scanning calorimetry (heating rate 5°C/min).
(11) Aripiprazole Anhydride Crystals B of the present invention have low hygroscopicity. For example, Aripiprazole Anhydride Crystals B of the present invention maintain a water content of 0.4% or less after 24 hours inside a dessicator set at a temperature of 60°C and a humidity of 100%. Well-known methods of measuring water content can be used as long as they are methods commonly used for measuring the water content of crystals. For example, a method such as the Karl Fischer method can be used.
(12) When the small particle size is required for the formulation such as tablet and other solid dose formulations including for example flashmelt formulations, the mean particle size is preferably 50 μm or less.

Process for Manufacturing Anhydride B

In case of the formulation for which small particle size (less than 50 μm) is required, the milling is necessary for the preparation. However, when a large amount of Conventional Aripiprazole Anhydride or Anhydride Crystals B having large particle size is milled, the milled substances adhere with each other in the milling machine. Accordingly, there is a disadvantage that it is difficult to industrially prepare Aripiprazole Anhydride Crystals B having small particle size.
Under the circumstances, the inventors of the present invention have found that Conventional hydrate can be easily milled, and Aripiprazole Anhydride B having small particle size can be obtained in high yield with good-operability by heating the milled hydrate A thus obtained.
The Aripiprazole Anhydride Crystals B of the present invention are prepared for example by heating the aforementioned Aripiprazole Hydrate A at 90-125°C. The heating time is generally about 3-50 hours, but cannot be stated unconditionally since it differs depending on heating temperature. The heating time and heating temperature are inversely related, so that for example the heating time will be longer the lower the heating temperature, and shorter the higher the heating temperature. Specifically, if the heating temperature of Aripiprazole Hydrate A is 100°C, the heating time should normally be 18 hours or more or preferably about 24 hours. If the heating temperature of Aripiprazole Hydrate A is 120°C, on the other hand, the heating time can be about 3 hours. The Aripiprazole Anhydride Crystals B of the present invention can be prepared with certainty by heating Aripiprazole Hydrate A for about 18 hours at 100°C, and then heating it for about 3 hours at 120°C. The Aripiprazole Anhydride Crystals B of the present invention can also be obtained if the heating time is extended still further, but this may not be economical.
When small particle size is not required for the formulation, e.g., when drug substance is being manufactured for injectable or oral solution formulations, Aripiprazole Anhydride Crystal B can be also obtained the following process.
The inventors also discovered that it is possible to obtain aripiprazole anhydride crystals by heating conventional aripiprazole hydrate or conventional aripiprazole anhydride crystals to a specific temperature but this process does not yield Anhydride B crystalline substance suitable for commercial use in the formulation of solid oral dose formulations.
Furthermore, the Aripiprazole Anhydride Crystals B of the present invention are prepared for example by heating conventional aripiprazole anhydride crystals at 90-125°C. The heating time is generally about 3-50 hours, but cannot be stated unconditionally since it differs depending on heating temperature. The heating time and heating temperature are inversely related, so that for example the heating time will be longer the lower the heating temperature, and shorter the higher the heating temperature.
Specifically, if the heating temperature of the aripiprazole anhydride crystals is 100°C, the heating time can be about 4 hours, and if the heating temperature is 120°C the heating time can be about 3 hours.
In addition to Aripiprazole Hydrate A and Aripiprazole Anhydride Crystals B mentioned above, the present invention provides Aripiprazole Anhydride Crystals C to G as follows.

1. The present invention relates to aripiprazole anhydride crystals (hereinafter referred to as "type C crystals of aripiprazole anhydride") having the following physicochemical properties (1) to (5) :

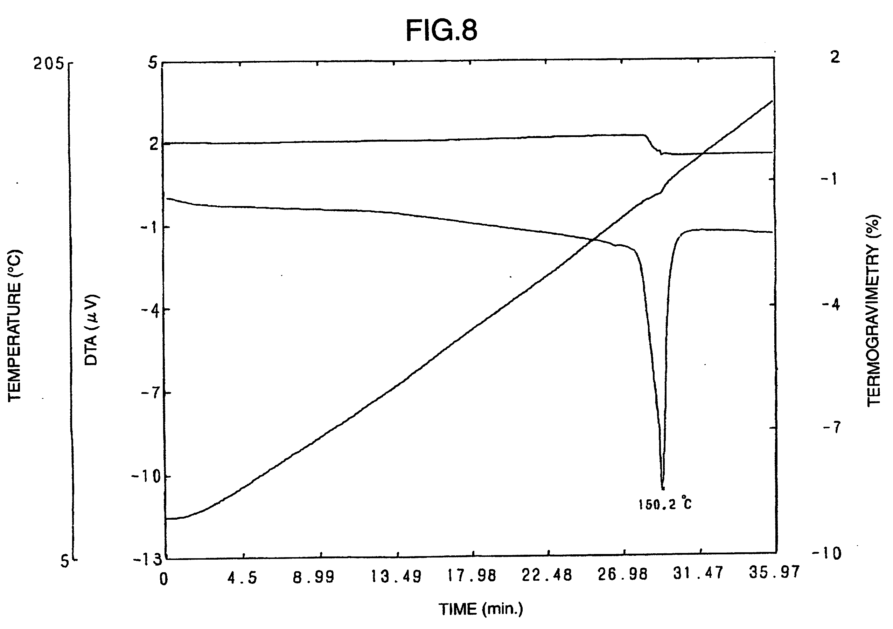

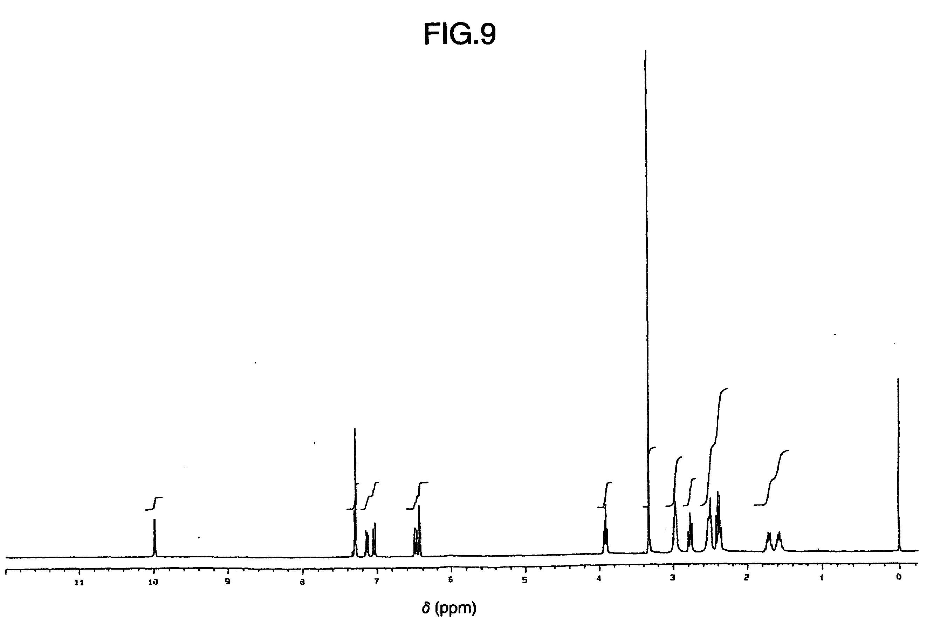

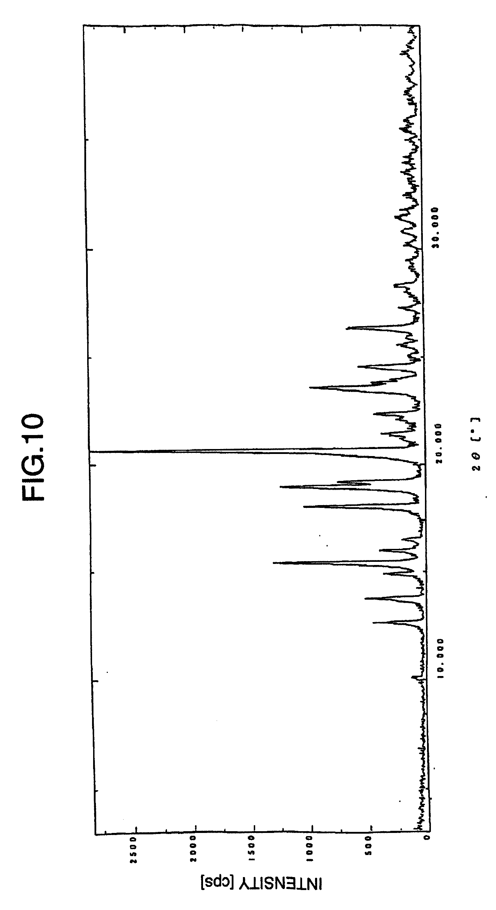

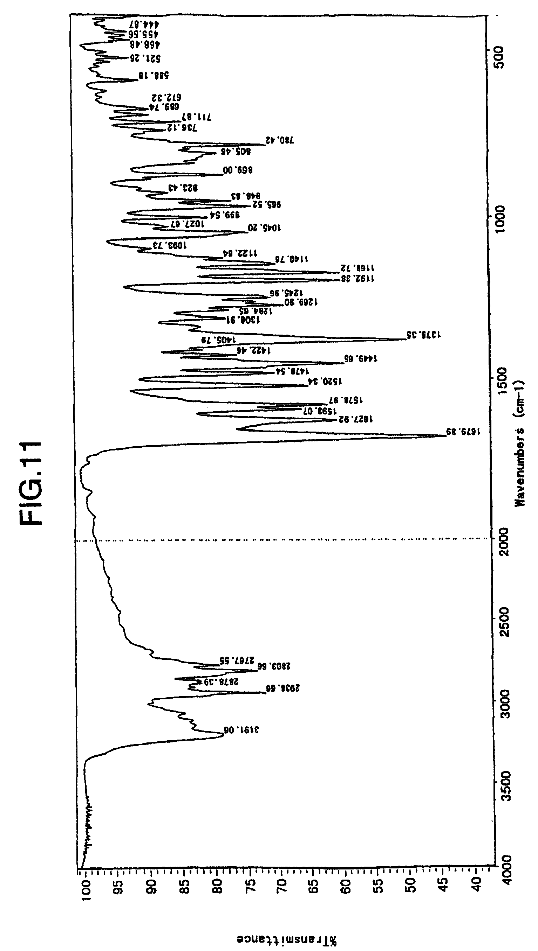

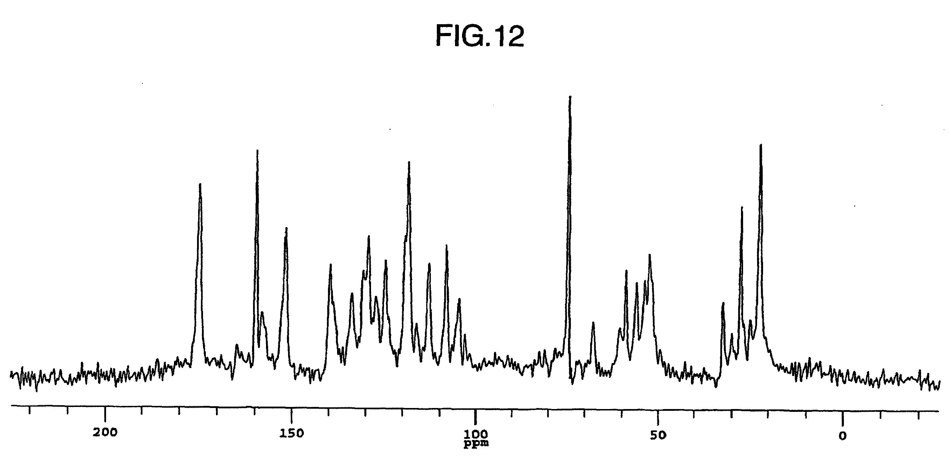

(1) an endothermic curve which is substantially identical to the thermogravimetric/ differential thermal analysis (heating rate: 5°C/min.) endothermic curve shown in Figure 8;
(2) an $^1$H-NMR spectrum which is substantially identical to the $^1$H-NMR spectrum (DMSO-d$_6$, TMS) shown in Figure 9;
(3) a powder X-ray diffraction spectrum which is substantially identical to the powder X-ray diffraction spectrum shown in Figure 10;
(4) an IR spectrum which is substantially identical to the IR (KBr) shown in Figure 11; and
(5) a solid $^{13}$C-NMR spectrum which is substantially identical to the solid $^{13}$C-NMR spectrum shown in Figure 12.

2. The present invention relates to aripiprazole anhydride crystals (hereinafter referred to as "type D crystals of aripiprazole anhydride") having the following physicochemical properties (6) to (10) :

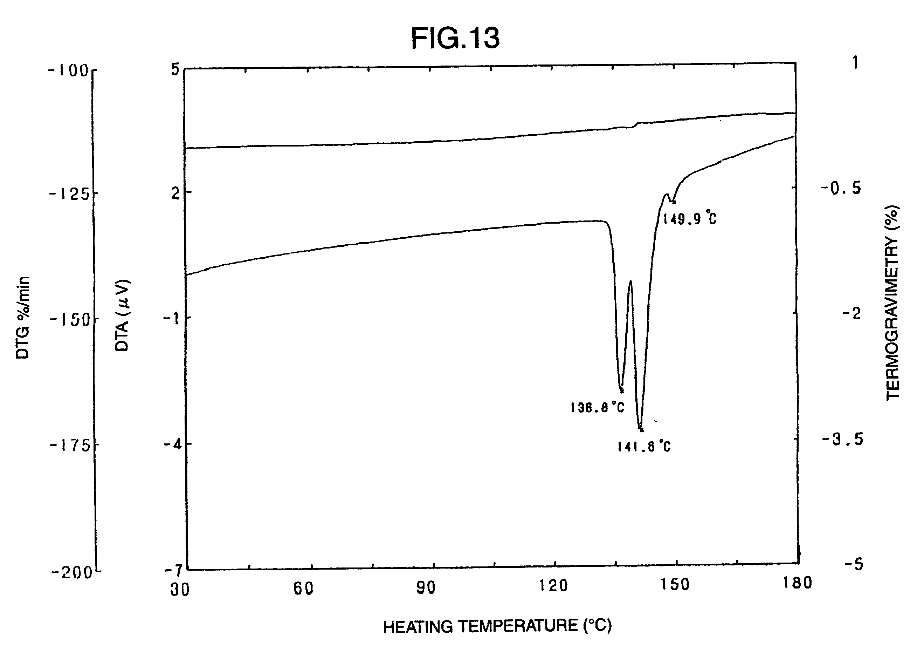

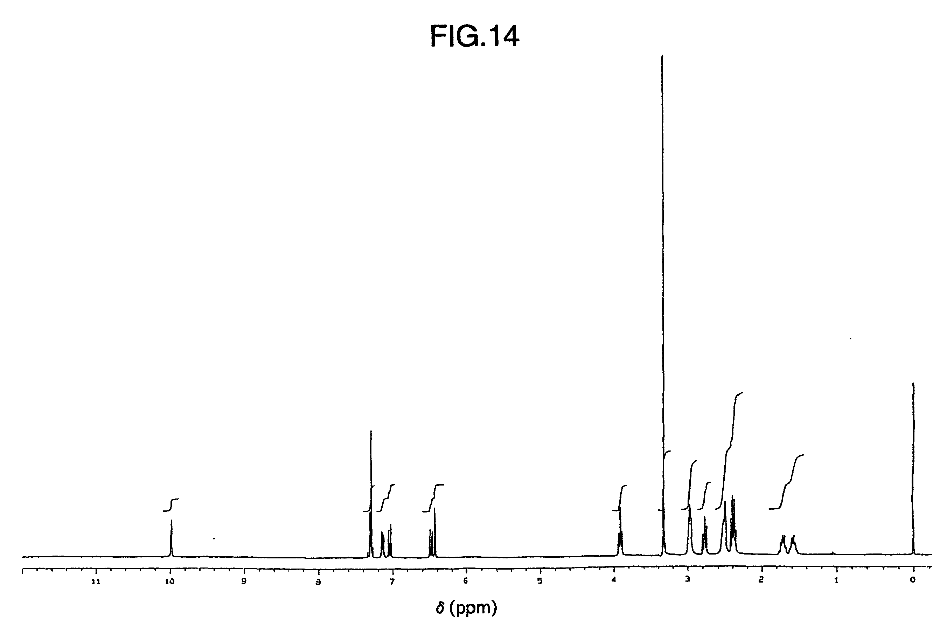

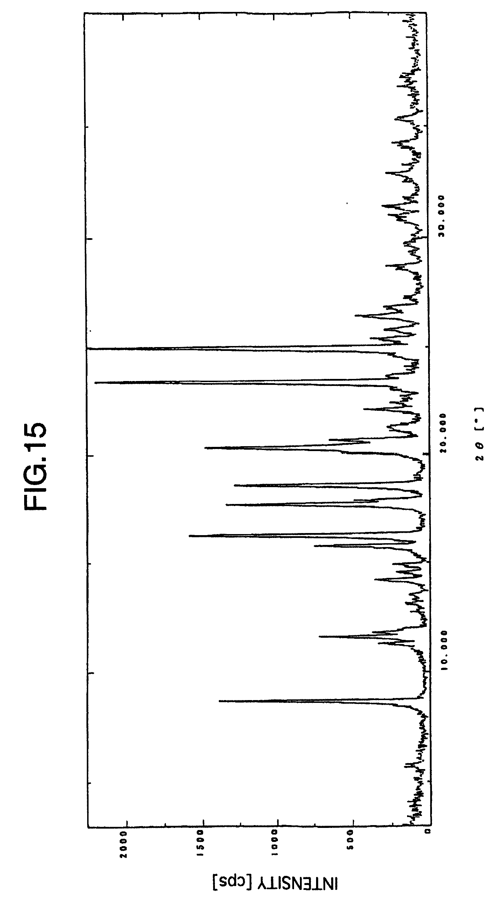

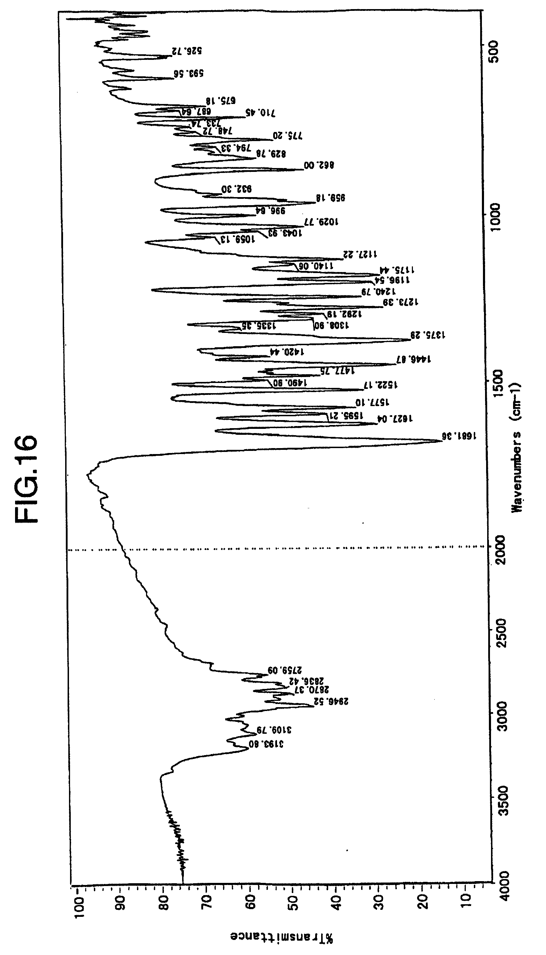

(6) an endothermic curve which is substantially identical to the thermogravimetric/ differential thermal analysis (heating rate: 5°C/min.) endothermic curve shown in Figure 13;
(7) an $^1$H-NMR spectrum which is substantially identical to the $^1$H-NMR spectrum (DMSO-d$_6$, TMS) shown in Figure 14;
(8) a powder X-ray diffraction spectrum which is substantially identical to the powder X-ray diffraction spectrum shown in Figure 15;
(9) an IR spectrum which is substantially identical to the IR (KBr) shown in Figure 16; and

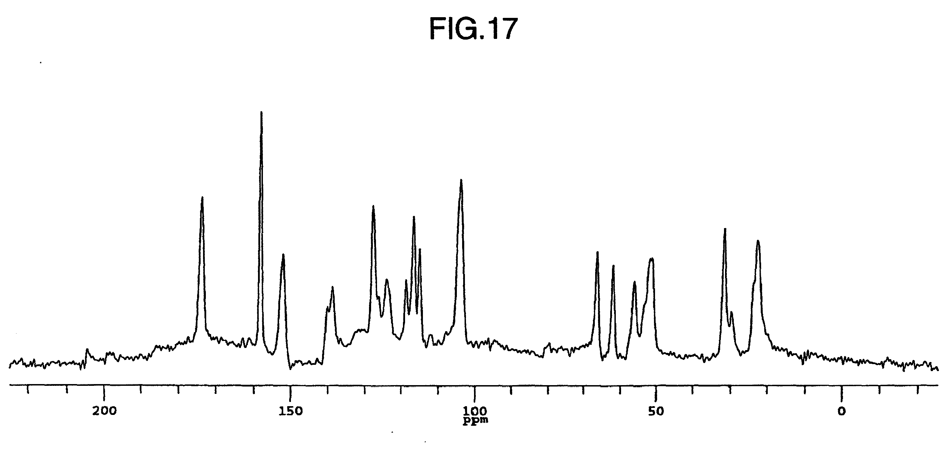

(10) a solid $^{13}$C-NMR spectrum which is substantially identical to the $^{13}$C-NMR spectrum shown in Figure 17.

3. The present invention relates to aripiprazole anhydride crystals (hereinafter referred to as "type E crystals of aripiprazole anhydride") having the following physicochemical properties (11) to (14):

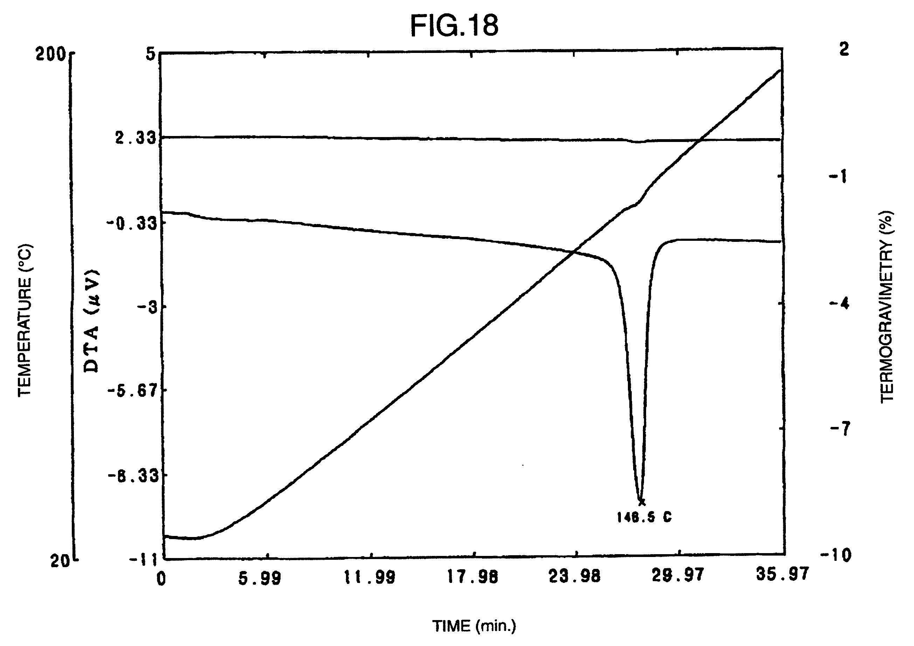

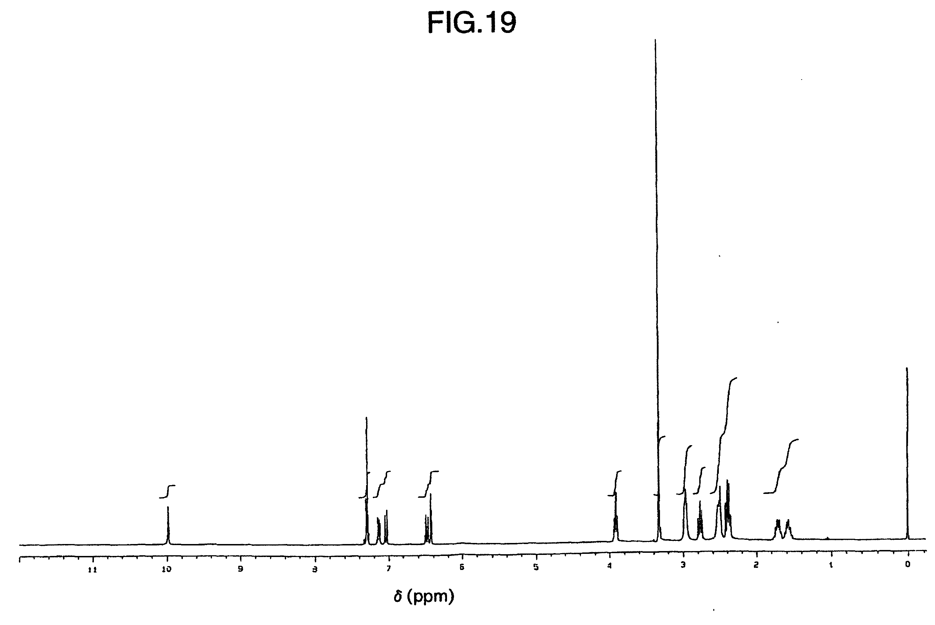

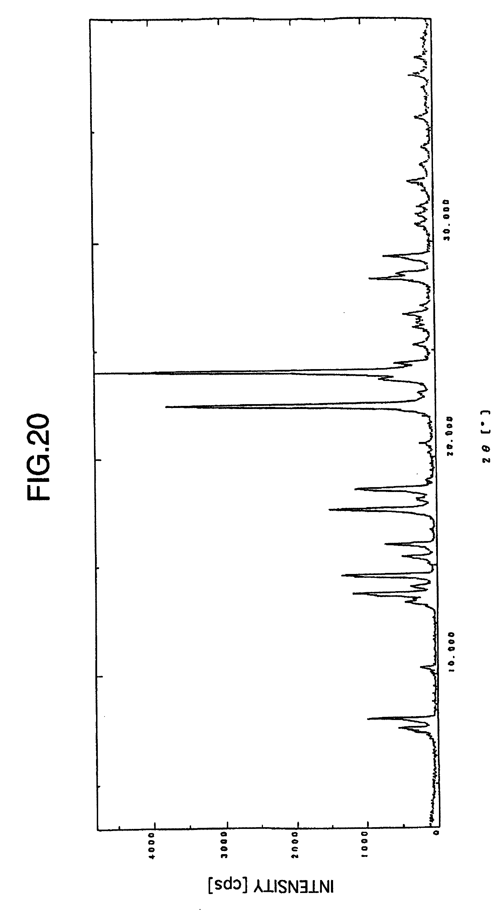

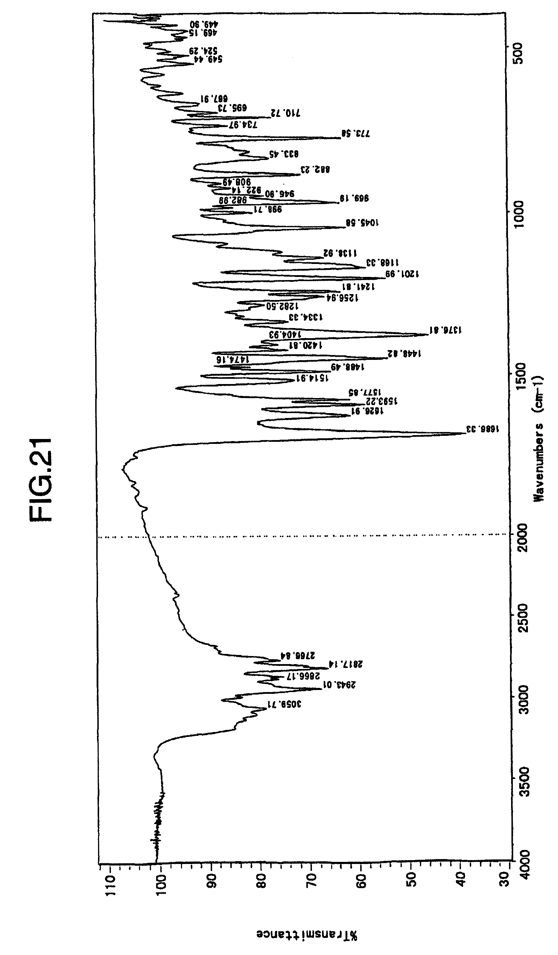

(11) an endothermic curve which is substantially identical to the thermogravimetric/ differential thermal analysis (heating rate: 5°C/min.) endothermic curve shown in Figure 18;
(12) an $^1$H-NMR spectrum which is substantially identical to the $^1$H-NMR spectrum (DMSO-d$_6$, TMS) shown in Figure 19;
(13) a powder X-ray diffraction spectrum which is substantially identical to the powder X-ray diffraction spectrum shown in Figure 20; and
(14) an IR spectrum which is substantially identical to the IR (KBr) shown in Figure 21.

4. The present invention relates to aripiprazole anhydride crystals (hereinafter referred to as "type F crystals of aripiprazole anhydride") having the following physicochemical properties (15) to (18):

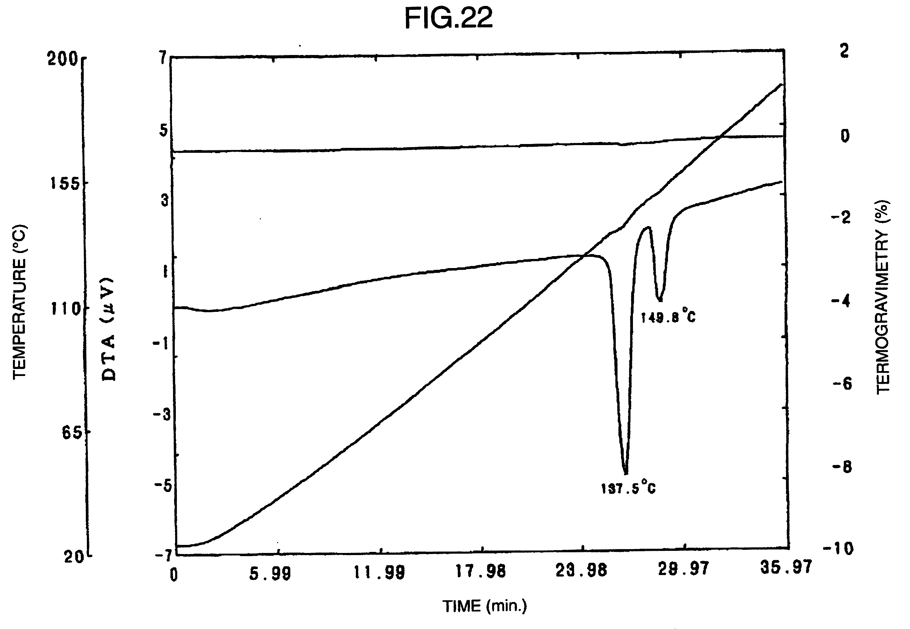

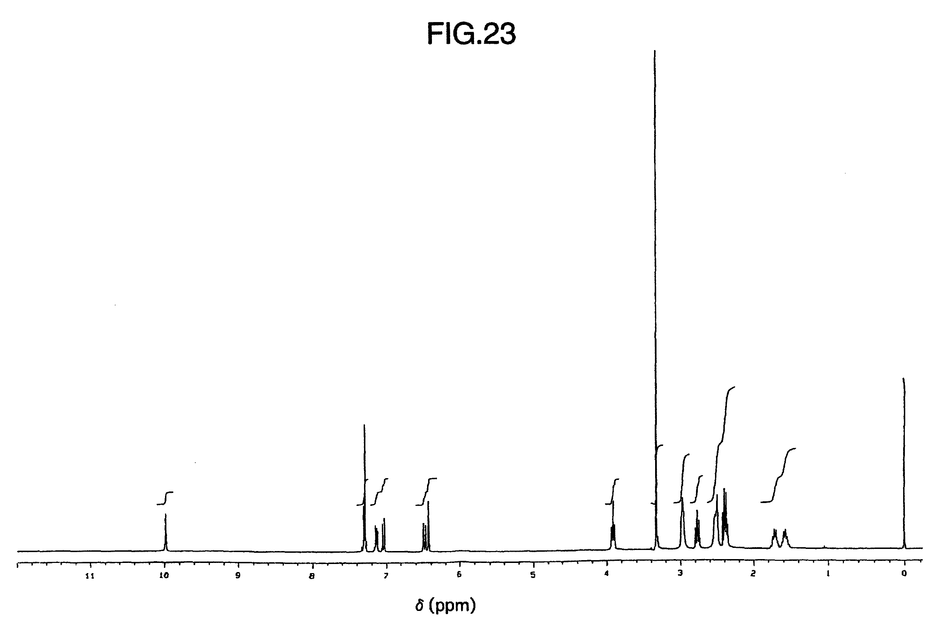

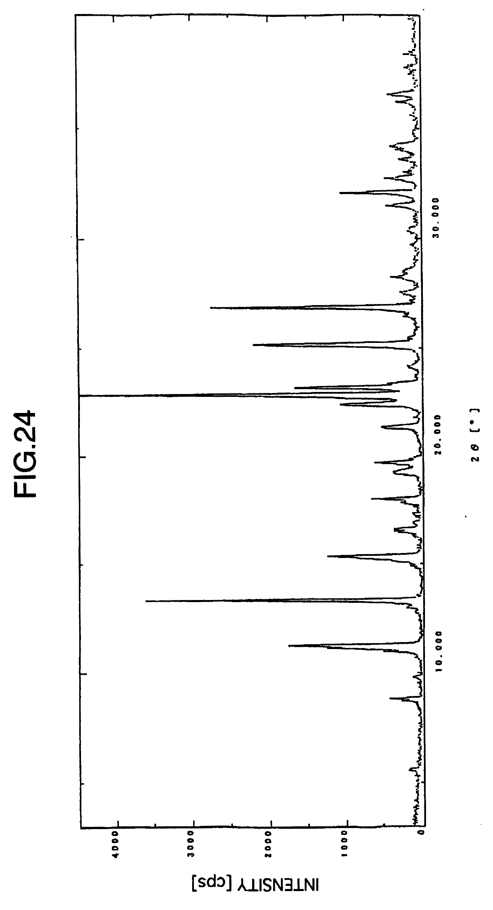

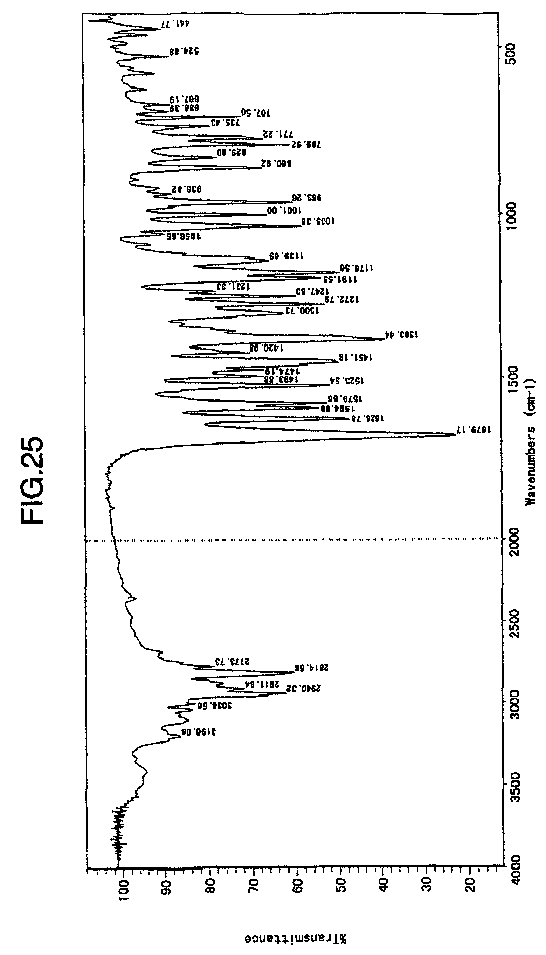

(15) an endothermic curve which is substantially identical to the thermogravimetric/ differential thermal analysis (heating rate: 5°C/min.) endothermic curve shown in Figure 22;
(16) an $^1$H-NMR spectrum which is substantially identical to the $^1$H-NMR spectrum (DMSO-d$_6$, TMS) shown in Figure 23;
(17) a powder X-ray diffraction spectrum which is substantially identical to the powder X-ray diffraction spectrum shown in Figure 24; and
(18) an IR spectrum which is substantially identical to the IR (KBr) shown in Figure 25.

5. The present invention relates a process for preparing aripiprazole anhydride crystals stated in the aforementioned item 1, characterized by heating aripiprazole anhydride crystals at a temperature being higher than 140°C and lower than 150°C.

6. The present invention relates a process for preparing aripiprazole anhydride crystals stated in the aforementioned item 2, characterized by recrystallizing from toluene.

7. The present invention relates to a process for preparing aripiprazole anhydride crystals stated in the aforementioned item 3, characterized by heating and dissolving aripiprazole anhydride crystals in acetonitrile, and cooling it.

8. The present invention relates to a process for preparing aripiprazole anhydride crystals stated in the aforementioned item 4, characterized by heating a suspension of aripiprazole anhydride crystals in acetone.

9. The present invention relates to a pharmaceutical composition containing at least one aripiprazole anhydride crystals selected from the group consisting of the aripiprazole anhydride crystals stated in the aforementioned item 1, the aripiprazole anhydride crystals stated in the aforementioned item 2, the aripiprazole anhydride crystals stated in the aforementioned item 3, the aripiprazole anhydride crystals stated in the aforementioned item 4, and the aripiprazole anhydride crystals stated in the after-mentioned item 10, together with pharmaceutically acceptable carriers.

10. The present invention relates to aripiprazole anhydride crystals (hereinafter referred to as "type G crystals of aripiprazole anhydride") having the following physicochemical properties (19) to (22):

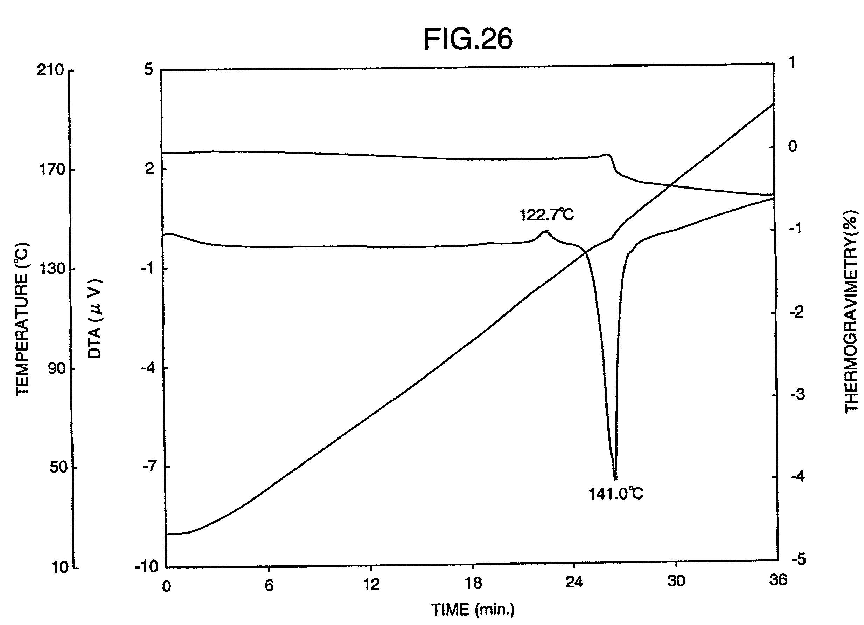

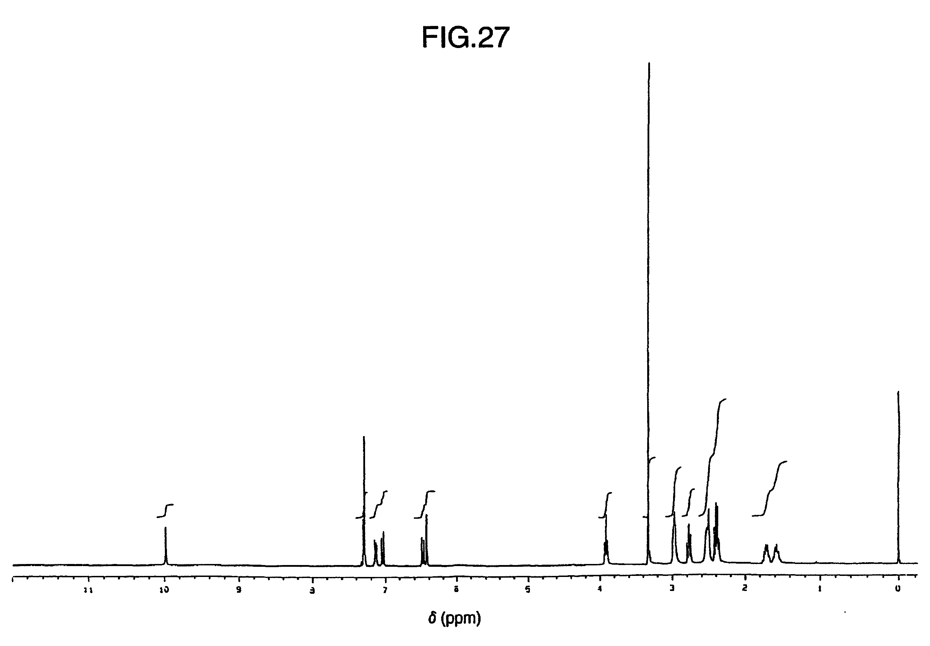

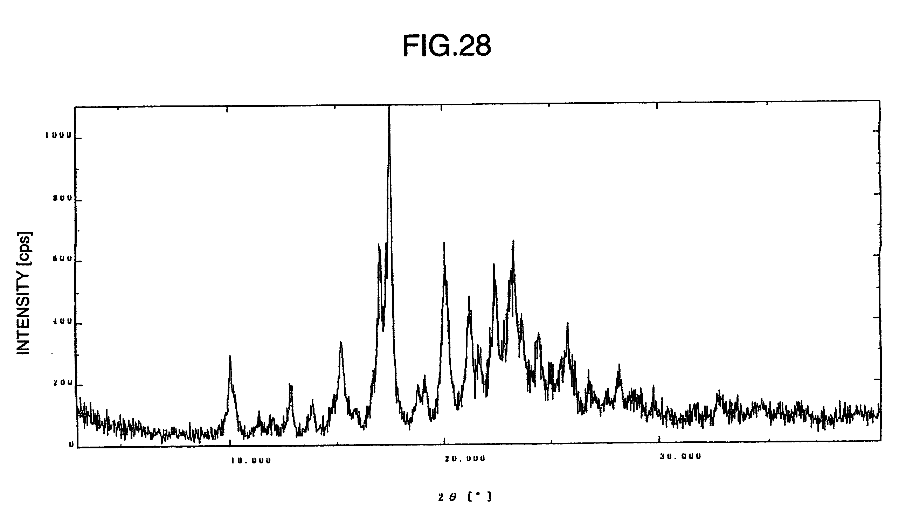

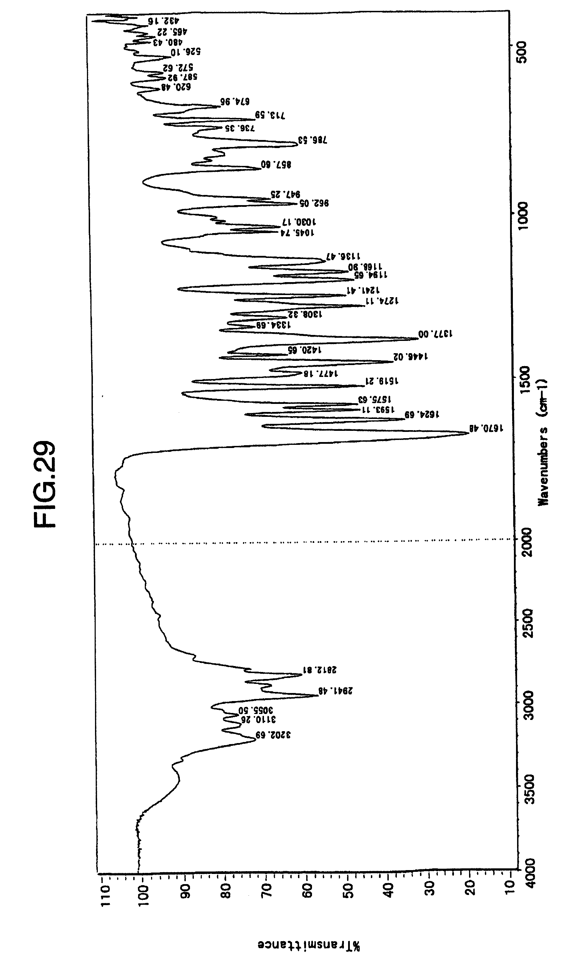

(19) an endothermic curve which is substantially identical to the thermogravimetric/differential thermal analysis (heating rate; 5°C/min.) endothermic curve shown Figure 26.
(20) an $^1$H-NMR spectrum which is substantially identical to the $^1$H-NMR spectrum (DMSO-d$_6$, TMS) shown in Figure 27.
(21) a power X-ray diffraction spectrum which is substantially identical to the power X-ray diffraction spectrum shown in Figure 28; and
(22) an IR spectrum which is substnatially identical to the IR (Kbr) shown in Figure 29.

11. The present invention relates to a process for preparing aripiprazole anhydride crystals stated in the aforementioned item 10, characterized by putting glassy state of Aripiprazole Anhydride in a sealed vessel and keeping it at room temperature for at least 2 weeks.

12. The present invention relates to a process for the preparation of granules, characterized by wet granulating conventional Aripiprazole Anhydride Crystals or Aripiprazole Anhydride Crystals B, C, D, E, F or G, drying the obtained granules at 70 to 100°C and sizing it, then drying the sized granules at 70 to 100°C again.

13. The present invention relates to a process for the pharmaceutical solid oral preparation, characterized by drying a pharmaceutical solid oral preparation comprising conventional Aripiprazole Anhydride Crystals or Aripiprazole Anhydride Crystals B, C, D, E, F or G, and one or more pharmaceutically acceptable carriers at 70 to 100°C.

14. The present invention relates to a pharmaceutical solid oral preparation comprising Aripiprazole Anhydride Crystals B, C, D, E, F or G and one or more pharmaceutically acceptable carriers, wherein said pharmaceutical solid oral preparation has at least one dissolution rate selected from the group consisting 60% or more at pH 4.5 after 30 minutes, 70% or more at pH 4.5 after 60 minutes, and 55% or more at pH 5.0 after 60 minutes.

15. The present invention relates to a pharmaceutical solid oral preparation having at least one dissolution rate selected from the group consisting 60% or more at pH 4.5 after 30 minutes, 70% or more at pH 4.5 after 60 minutes, and 55% or more at pH 5.0 after 60 minutes.

16. The present invention relates to a pharmaceutical solid oral preparation obtained by wet granulating conventional Aripiprazole Anhydride Crystals, drying the obtained granules at 70 to 100°C and sizing it, then drying the sized granules at 70 to 100°C again, and the pharmaceutical solid oral preparation has at least one dissolution rate selected from the group consisting 60% or more at pH 4.5 after 30 minutes, 70% or more at pH 4.5 after 60 minutes, and 55% or more at pH 5.0 after 60 minutes.

17. The present invention relates to a pharmaceutical solid oral preparation obtained by drying a pharmaceutical solid oral preparation comprising conventional Aripiprazole Anhydride Crystals and one or more pharmaceutically acceptable carriers at 70 to 100°C, and the pharmaceutical solid oral preparation has at least one dissolution rate selected from the group consisting 60% or more at pH 4.5 after 30 minutes, 70% or more at pH 4.5 after 60 minutes, and 55% or more at pH 5.0 after 60 minutes.

18. The present invention relates to a process for the preparation of granules, characterized by wet granulating conventional Aripiprazole Hydrate Crystals, drying the obtained granules at 70 to 100°C and sizing it, then drying the sized granules at 70 to 100°C again.

19. The present invention relates to a process for the pharmaceutical solid oral preparation, characterized by drying a pharmaceutical solid oral preparation comprising conventional Aripiprazole Hydrate Crystals and one or more pharmaceutically acceptable carriers at 70 to 100°C.

20. The present invention relates to a pharmaceutical solid oral preparation obtained by wet granulating conventional Aripiprazole Hydrate Crystals, drying the obtained granules at 70 to 100°C and sizing it, then drying the sized granules at 70 to 100°C again, and the pharmaceutical solid oral preparation has at least one dissolution rate selected from the group consisting 60% or more at pH 4.5 after 30 minutes, 70% or more at pH 4.5 after 60 minutes, and 55% or more at pH 5.0 after 60 minutes.

21. The present invention relates to a pharmaceutical solid oral preparation obtained by drying a pharmaceutical solid oral preparation comprising conventional Aripiprazole Hydrate Crystals and one or more pharmaceutically acceptable carriers at 70 to 100°C, and the pharmaceutical solid oral preparation has at least one dissolution rate selected from the group consisting 60% or more at pH 4.5 after 30 minutes, 70% or more at pH 4.5 after 60 minutes, and 55% or more at pH 5.0 after 60 minutes.

The Type C to F crystals of aripiprazole anhydride of the present invention correspond to the Type-III to VI crystals of aripiprazole anhydride disclosed in JP-2001-348276.

Type C crystals of aripiprazole anhydride

Type C crystals of aripiprazole anhydride of the present invention have the following physicochemical properties (1) to (5):

(1) an endothermic curve which is substantially identical to the thermogravimetric/ differential thermal analysis (heating rate: 5°C/min.) endothermic curve shown in Figure 8, more particularly, it has an endothermic peak around 150.2°C;

(2) an $^1$H-NMR spectrum which is substantially identical to the $^1$H-NMR spectrum (DMSO-d$_6$, TMS) shown in Figure 9. Specifically, it has characteristic peaks at 1.55 - 1.63 ppm (m, 2H), 1.68 - 1.78 ppm (m, 2H), 2.35 - 2.46 ppm (m, 4H), 2.48 - 2.56 ppm (m, 4H + DMSO), 2.78 ppm (t, J=7, 4 Hz, 2H), 2.97 ppm (brt, J=4.6 Hz, 4H), 3.92 ppm (t, J=6.3 Hz, 2H), 6.43 ppm (d, J=2.4Hz, 1H), 6.49 ppm (dd, J=8.4 Hz, J=2.4 Hz, 1H), 7.04 ppm (d, J=8.1 Hz, 1H), 7.11 - 7.17 ppm (m, 1H), 7.28 - 7.32 ppm (m, 2H) and 10.00 ppm (s, 1H);

(3) a powder X-ray diffraction spectrum which is substantially identical to the powder X-ray diffraction spectrum shown in Figure 10. Specifically, it has characteristic peaks at $2\theta$ = 12.6°, 13.7°, 15.4°, 18.1°, 19.0°, 20.6°, 23.5° and 26.4°;

(4) an IR spectrum which is substantially identical to the IR (KBr) spectrum shown in Figure 11. Specifically, it has clear infrared absorption bands at 2939, 2804, 1680, 1375 and 780 cm$^{-1}$; and

(5) a solid $^{13}$C-NMR spectrum which is substantially identical to the solid $^{13}$C-NMR spectrum shown in Figure 12, specifically, it has characteristic peaks at 32.8 ppm, 60.8 ppm, 74.9 ppm, 104.9 ppm, 152.2 ppm, 159.9 ppm and 175.2 ppm.

Preparation method of type C crystals of aripiprazole anhydride

Type C crystals of aripiprazole anhydride of the present invention is prepared, for example by heating an aripiprazole anhydride at a temperature of higher than 140°C and lower than 150°C.

Aripiprazole anhydride used as the raw material may be conventional aripiprazole anhydride crystals, for example, type-I crystals of aripiprazole anhydride, type-II crystals of aripiprazole anhydride crystals and the like, and these anhydrides may be either purified products or crude materials. Alternatively, type B crystals of aripiprazole anhydride, type D crystals of aripiprazole anhydride, type E crystals of aripiprazole anhydride, type F crystals of aripiprazole anhydride, or type G crystals of aripiprazole anhydride being prepared in the present invention can be used as the raw material of aripiprazole anhydrides. These aripiprazole anhydrides can be used singly or in combination of at least 2 kinds thereof.

Heating temperature is generally higher than 140°C and lower than 150°C, preferably at 142 - 148°C, and heating time is generally for 15 minutes to 3 hours, preferably for 30 minutes to 1 hour.

When, an aripiprazole anhydride is heated at the above-mentioned temperature, then type C crystals of aripiprazole anhydride are formed.

Thus obtained type C crystals of aripiprazole anhydride can be isolated and purified by well-known methods. For example, after heating the aripiprazole anhydride at the above-mentioned temperature, and by cooling to a room temperature, then type C crystals of aripiprazole anhydride, having 100 % of purity can be obtained.

Type D crystals of aripiprazole anhydride

Type D crystals of aripiprazole anhydride of the present invention have the following physicochemical properties (6) to (10):

(6) an endothermic curve which is substantially identical to the thermogravimetric/ differential thermal analysis (heating rate: 5°C/min.) endothermic curve shown in Figure 13; more particularly, it has an endothermic peak around 136.8 °C and 141.6 °C;

(7) an $^1$H-NMR spectrum which is substantially identical to the $^1$H-NMR spectrum (DMSO-$d_6$, TMS) shown in Figure 14. Specifically, it has characteristic peaks at 1.55 - 1.63 ppm (m, 2H), 1.68 - 1.78 ppm (m, 2H), 2.35 - 2.46 ppm (m, 4H), 2.48 - 2.56 ppm (m, 4H + DMSO), 2.78 ppm (t, J=7, 4 Hz, 2H), 2.97 ppm (brt, J=4.6 Hz, 4H), 3.92 ppm (t, J=6.3 Hz, 2H), 6.43 ppm (d, J=2.4Hz, 1H), 6.49 ppm (dd, J=8.4 Hz, J=2.4 Hz, 1H), 7.04 ppm (d, J=8.1 Hz, 1H), 7.11 - 7.17 ppm (m, 1H), 7.28 - 7.32 ppm (m, 2H) and 10.00 ppm (s, 1H);

(8) a powder X-ray diffraction spectrum which is substantially identical to the powder X-ray diffraction spectrum shown in Figure 15. Specifically, it has characteristic peaks at $2\theta$ = 8.7°, 11.6°, 16.3°, 17.7°, 18.6°, 20.3°, 23.4° and 25.0°;

(9) an IR spectrum which is substantially identical to the IR (KBr) spectrum shown in Figure 16. Specifically, it has clear infrared absorption bands at 2946, 1681, 1375, 1273, 1175 and 862 cm$^{-1}$; and

(10) a solid $^{13}$C-NMR spectrum which is substantially identical to the solid $^{13}$C-NMR spectrum shown in Figure 17, specifically, it has characteristic peaks at 32.1 ppm, 62.2 ppm, 66.6 ppm, 104.1 ppm, 152.4 ppm, 158.4 ppm, and 174.1 ppm.

Preparation method of type D crystals of aripiprazole anhydride

Type D crystals of aripiprazole anhydride of the present invention is prepared, for example, by recrystallization of aripiprazole anhydride from toluene. Specifically, an aripiprazole anhydride is added to toluene, further heated and dissolved, then thus obtained solution is cooled. By such procedures, type D crystals of aripiprazole anhydride of the present invention is separated out as crystals in toluene.

Aripiprazole anhydride to be used as the raw materials may be conventional aripiprazole anhydride, for example type-I crystals of aripiprazole anhydride, type-II crystals of aripiprazole anhydride and the like, and these anhydrides may be either purified products or crude materials. Alternatively, type B crystals of aripiprazole anhydride, type C crystals of aripiprazole anhydride, type E crystals of aripiprazole anhydride, type F crystals of aripiprazole anhydride, or type G crystals of aripiprazole anhydride being prepared in the present invention can be used as the raw material for aripiprazole anhydrides. These aripiprazole anhydrides can be used singly or in combination of at least 2 kinds thereof.

When the solution obtained by heating and dissolving is cooled, type D crystals of aripiprazole may be added as a seed crystal to said solution. Further, the seed crystal may be formed by cooling gradually said solution being obtained by heating and dissolving. In the presence of the seed crystal, type D crystals of aripiprazole anhydride may be separated out.

Thus separated out type D crystals of aripiprazole anhydride can be isolated and purified in accordance with well-known methods. By such procedures, type D crystals of aripiprazole anhydride, having the purity of 100 % can be obtained.

Type E crystals of aripiprazole anhydride

Type E crystals of aripiprazole anhydride of the present invention have the following physicochemical properties (11) to (14):

(11) an endothermic curve which is substantially identical to the thermogravimetric/ differential thermal analysis (heating rate: 5°C/min.) endothermic curve shown in Figure 18, specifically, it has an endothermic peak around 146.5°C;

(12) an $^1$H-NMR spectrum which is substantially identical to the $^1$H-NMR spectrum (DMSO-d$_6$, TMS) shown in Figure 19. Specifically, it has characteristic peaks at 1.55 - 1.63 ppm (m, 2H), 1.68 - 1.78 ppm (m, 2H), 2.35 - 2.46 ppm (m, 4H), 2.48 - 2.56 ppm (m, 4H + DMSO), 2.78 ppm (t, J=7, 4 Hz, 2H), 2.97 ppm (brt, J=4.6 Hz, 4H), 3.92 ppm (t, J=6.3 Hz, 2H), 6.43 ppm (d, J=2.4Hz, 1H), 6.49 ppm (dd, J=8.4 Hz, J=2.4 Hz, 1H), 7.04 ppm (d, J=8.1 Hz, 1H),7.11 - 7.17 ppm (m, 1H), 7.28 - 7.32 ppm (m, 2H) and 10.00 ppm (s, 1H);

(13) a powder X-ray diffraction spectrum which is substantially identical to the powder X-ray diffraction spectrum shown in Figure 20. Specifically, it has characteristic peaks at 2θ = 8.0°, 13.7°, 14.6°, 17.6°, 22.5° and 24.0°; and

(14) an IR spectrum which is substantially identical to the IR (KBr) spectrum shown in Figure 21. Specifically, it has clear infrared absorption bands at 2943, 2817,1686, 1377, 1202, 969 and 774 cm$^{-1}$.

Preparation method of type E crystals of aripiprazole anhydride

Type E crystals of aripiprazole anhydride of the present invention is prepared, for example by recrystallization of the aripiprazole anhydride from acetonitrile. Specifically, by adding a well-known aripiprazole anhydride to acetonitrile, heating and dissolving, then the solution thus obtained may be cooled. In accordance with such procedures, type E crystals of aripiprazole anhydride of the present invention are separated out in the acetonitrile.

When a conventional aripiprazole anhydride is added to acetonitrile, type-I crystals of aripiprazole anhydride, type-II crystals of aripiprazole anhydride and type D crystals of aripiprazole anhydride are separated out, other than type E crystals of aripiprazole anhydride. Plate crystals being separated out from the acetonitrile solution at 70°C are type-I crystals, type-II crystals and type D crystals, while type E crystals are precipitated out as needle crystals. When the acetonitrile solution after separated out of these crystals is heated again (for example, heated at over 75°C), the plate crystals (type-I crystals, type-II crystals and type D crystals) are quickly dissolved, on the contrary, the needle form crystals (type E crystals) do not dissolved. Additionally, when the acetonitrile solution is cooled again, then needle form crystals (type E crystals) are further separated out around the needle form crystals (type E crystals) previously precipitated as the seed crystals. Thus, type E crystals of aripiprazole anhydride can be precipitated in the acetonitrile solution.

Aripiprazole anhydrides used as the raw materials may be conventional aripiprazole anhydrides, for example any one of type-I crystals of aripiprazole anhydride and type-II crystals of aripiprazole anhydride and the like, and these anhydrides may be either purified products or crude materials. Alternatively, type B crystals of aripiprazole anhydride, type C crystals of aripiprazole anhydride, type D crystals of aripiprazole anhydride, type F crystals of aripiprazole anhydride, or type G crystals of aripiprazole anhydride can be used as the raw materials for aripiprazole anhydrides. These aripiprazole anhydrides can be used singly or in combination of at least 2 kinds thereof.

When the acetonitrile solution obtained by heating (heating and dissolving) is cooled, the type E crystals of aripiprazole may be added as a seed crystal to said solution. Further, the seed crystal may be formed by cooling gradually said acetonitrile solution which was obtained by heating.

Thus separated out type E crystals of aripiprazole anhydride can be isolated and purified in accordance with well-known methods. By such procedures, type E crystals of aripiprazole anhydride, having the purity of 100 % can be obtained.

Type F crystals of aripiprazole anhydride

Type F crystals of aripiprazole anhydride of the present invention have the following physicochemical properties (15) to (18):

(15) an endothermic curve which is substantially identical to the thermogravimetric/ differential thermal analysis (heating rate: 5°C/min.) endothermic curve shown in Figure 22, specifically, it has an endothermic peaks around 137.5°C and 149.8°C;

(16) an $^1$H-NMR spectrum which is substantially identical to the $^1$H-NMR spectrum (DMSO-$d_6$, TMS) shown in Figure 23. Specifically, it has characteristic peaks at 1.55 - 1.63 ppm (m, 2H), 1.68 - 1.78 ppm (m, 2H), 2.35 - 2.46 ppm (m, 4H), 2.48 - 2.56 ppm (m, 4H + DMSO), 2.78 ppm (t, J=7, 4 Hz, 2H), 2.97 ppm (brt, J=4.6 Hz, 4H), 3.92 ppm (t, J=6.3 Hz, 2H), 6.43 ppm (d, J=2.4Hz, 1H), 6.49 ppm (dd, J=8.4 Hz, J=2.4 Hz, 1H), 7.04 ppm (d, J=8.1 Hz, 1H), 7.11 - 7.17 ppm (m, 1H), 7.28 - 7.32 ppm (m, 2H) and 10.00 ppm (s, 1H);

(17) a powder X-ray diffraction spectrum which is substantially identical to the powder X-ray diffraction spectrum shown in Figure 24. Specifically, it has characteristic peaks at 2θ = 11.3°, 13.3°, 15.4°, 22.8°, 25.2° and 26.9°, and

(18) Having an IR spectrum which is substantially identical to the IR (KBr) spectrum shown in Figure 25. Specifically, it has clear infrared absorption bands at 2940, 2815,1679, 1383, 1273, 1177, 1035, 963 and 790 cm$^{-1}$.

Preparation method of type F crystals of aripiprazole anhydride

Type F crystals of aripiprazole anhydride of the present invention is prepared, for example by suspending an aripiprazole anhydride in acetone, and thus obtained acetone suspension is heated.

Aripiprazole anhydrides used as the raw materials may be conventional aripiprazole anhydride, for example any one of type-I crystals of aripiprazole anhydride and type-II crystals of aripiprazole anhydride and the like, and these anhydrides may be either purified products or crude materials. Alternatively, type B crystals of aripiprazole anhydride, type C crystals of aripiprazole anhydride, type D crystals of aripiprazole anhydride, type E crystals of aripiprazole anhydride, or type G crystals of aripiprazole anhydride prepared in the present invention can be used as the raw materials for aripiprazole anhydrides. These aripiprazole anhydrides can be used singly or in combination of at least 2 kinds thereof.

Heating temperature of the acetone suspension may be generally about the boiling point of acetone, and heating time is generally 5 to 10 hours. When the acetone suspension is heated about the boiling point of acetone, then type F crystals of aripiprazole anhydride is formed, the crystals are isolated by filtration with heating. Isolation of the crystals may be carried out in accordance with well-known methods. By such procedures, type F crystals of aripiprazole anhydride, having the purity of 100 % can be obtained.

Type G crystals of aripiprazole anhydride

Type G crystals of aripiprazole anhydride of the present invention have the following physicochemical properties (19) to (22):

(19) an endothermic curve which is substantially identical to the thermogravimetric/ differential thermal analysis (heating rate: 5°C/min.) endothermic curve shown in Figure 26, more particularly, it has an endothermic peak around 141.0°C and an exothermic peak around 122.7°C;

(20) an $^1$H-NMR spectrum which is substantially identical to the $^1$H-NMR spectrum (DMSO-$d_6$, TMS) shown in Figure 27. Specifically, it has characteristic peaks at 1.55 - 1.63 ppm (m, 2H), 1.68 - 1.78 ppm (m, 2H), 2.35 - 2.46 ppm (m, 4H), 2.48 - 2.56 ppm (m, 4H + DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 6.49 ppm (dd, J=8.4 Hz, J=2.4 Hz, 1H), 7.04 ppm (d, J=8.1 Hz, 1H), 7.11 - 7.17 ppm (m, 1H), 7.28 - 7.32 ppm (m, 2H) and 10.00 ppm (s, 1H);

(21) a powder X-ray diffraction spectrum which is substantially identical to the powder X-ray diffraction spectrum shown in Figure 28. Specifically, it has characteristic peaks at 2θ = 10.1°, 12.8°, 15.2°, 17.0°, 17.5°, 19.1°, 20.1°, 21.2°, 22.4°, 23.3°, 24.5° and 25.8°; and

(22) an IR spectrum which is substantially identical to the IR (KBr) spectrum shown in Figure 29. Specifically, it has clear infrared absorption bands at 2942, 2813, 1670, 1625, 1377, 1195, 962 and 787 cm$^{-1}$.

Preparation method of type G crystals of aripiprazole anhydride

Type G crystals of aripiprazole anhydride of the present invention can be prepared, for example by putting glassy state of aripiprazole anhydride in a sealed vessel and leaving to stand it at room temperature for at least two weeks, preferably two weeks to six months. Further, glassy state of aripiprazole anhydride as starting material can be obtained by heating and melting aripiprazole anhydride at around 170°C, then cooling it to room temperature.

Aripiprazole anhydride used as the raw material may be well-known aripiprazole anhydride crystals, for example, any one of type-I crystals of aripiprazole anhydride and type-II crystals of aripiprazole anhydride and the like, and these anhydrides may be either purified products or crude materials. Alternatively, type B crystals of aripiprazole anhydride, type C crystals of aripiprazole anhydride, type D crystals of aripiprazole anhydride, type E crystals of aripiprazole anhydride, or type F crystals of aripiprazole anhydride being prepared in the present invention can be used as the raw material of aripiprazole anhydrides. These aripiprazole anhydrides can be used singly or in combination of at least 2 kinds thereof.

Thus obtained type G crystals of aripiprazole anhydride can be isolated and purified by well-known methods.

For example, glassy state of aripiprazole anhydride leave to stand according to the above-mentioned method, then type G crystals of aripiprazole anhydride, having 100% of purity can be obtained.

Type C crystals of aripiprazole anhydride, type D crystals of aripiprazole anhydride, type E crystals of aripiprazole anhydride, type F crystals of aripiprazole anhydride and type G crystals of aripiprazole anhydride of the present invention neither easily convert into hydrates thereof, nor substantially decrease the original solubility, even when they are stored for a long period of time.

In accordance with the present invention, methods for preparing aripiprazole anhydride crystals having high purity, which can apply in an industrial scale with a good repeatability is provided.

In accordance with the present invention, pharmaceutical compositions comprising aripiprazole anhydride crystals are provided, of which the solubility does not decrease, and of which the stability can keep excellent, even if they are stored for long time.

The aripiprazole anhydride crystals which are the raw material for preparing the Aripiprazole Anhydride Crystals B to G of the present invention are prepared for example by Method a or b below.

"Method a": Process for Preparing Crude Aripiprazole Crystals

Conventional Aripiprazole Anhydride crystals are prepared by well-known methods, as described in Example 1 of Japanese Unexamined Patent Publication No. 191256/1990.

A suspension of 47 g of 7-(4-bromobutoxy)-3,4-dihydrocarbostyril, 35 g of sodium iodide with 600 ml of acetonitrile was refluxed for 30 minutes. To this suspension was added 40 g of 1-(2,3-dichlorophenyl)piperazine and 33 ml of triethylamine and the whole mixture was further refluxed for 3 hours. After the solvent was removed by evaporation, the residue thus obtained was dissolved in chloroform, washed with water then dried with anhydrous magnesium sulfate. The solvent was removed by evaporation, and the residue thus obtained was recrystallized from ethanol twice, to yield 57.1 g of 7-{4-[4-(2,3-dichlorophenyl)-1-piperazinyl]butoxy}-3,4-dihydrocarbostyril.

Colorless flake crystals
Melting point: 139.0-139.5°C

"Method b": Process for Preparing Conventional Anhydride

The Method b is described in the Proceedings of the 4th Japanese-Korean Symposium on Separation Technology (October 6-8, 1996).

Furthermore, the Aripiprazole Anhydride Crystals B of the present invention are prepared for example by heating conventional aripiprazole hydrate at 90-125°C. The heating time is generally about 3-50 hours, but cannot be stated unconditionally since it differs depending on heating temperature. The heating time and heating temperature are inversely related, so that for example the heating time will be longer the lower the heating temperature, and shorter the higher the heating temperature. Specifically, if the heating temperature of the aripiprazole hydrate is 100°C, the heating time can be about 24 hours, while if the heating temperature is 120°C, the heating time can be about 3 hours.

The aripiprazole hydrate which is the raw material for preparing the Aripiprazole Anhydride Crystals B of the present invention is prepared for example by Method c below.

"Method c": Process for Preparing Conventional Hydrate

Aripiprazole hydrate is easily obtained by dissolving the aripiprazole anhydride crystals obtained by Method a above in a hydrous solvent, and heating and then cooling the resulting solution. Using this method, aripiprazole hydrate is precipitated as crystals in the hydrous solvent.

An organic solvent containing water is usually used as the hydrous solvent. The organic solvent should be one which is miscible with water, such as for example an alcohol such as methanol, ethanol, propanol or isopropanol, a ketone such as acetone, an ether such as tetrahydrofuran, dimethylformamide, or a mixture thereof, with ethanol being particularly desirable. The amount of water in the hydrous solvent can be 10-25% by volume of the solvent, or preferably close to 20% by volume.

Medicinal Composition

A medicinal composition of the present invention will contain Aripiprazole Anhydride Crystals B, C, D, E, F and G in a pharmaceutically acceptable carrier or combination of carriers.

Carriers which are pharmaceutically acceptable include diluents and excipients generally used in pharmaceuticals, such as fillers, extenders, binders, moisturizers, disintegrators, surfactants, and lubricants.

The medicinal composition of the present invention may be formulated as an ordinary medicinal preparation, for example in the form of tablets, flashmelt tablets, pills, powder, liquid, suspension, emulsion, granules, capsules, suppositories or as an injection (liquid, suspension, etc.).

When a tablet formulation is used, a wide variety of carriers that are known in the field can be used. Examples include lactose, saccharose, sodium chloride, glucose, xylitol, mannitol, erythritol, sorbitol, urea, starch, calcium carbonate, kaolin, crystal cellulose, silic acid and other excipients; water, ethanol, propanol, simple syrup, glucose liquid, starch liquid, gelatin solution, carboxymethyl cellulose, shellac, methyl cellulose, potassium phosphate, polyvinyl pyrolidone and other binders; dried starch, sodium alginate, agar powder, laminaran powder, sodium bicarbonate, calcium carbonate, polyoxyethylene sorbitan fatty acid esters, sodium lauryl sulfate, monoglyceride stearate, starch, lactose and other disintegrators; saccharose, stearin, cacao butter, hydrogenated oil and other disintegration inhibitors; quaternary ammonium salt, sodium lauryl sulfate and other absorption promoters; glycerine, starch and other moisture retainers; starch, lactose, kaolin, bentonite, colloidal silic acid and other adsorbents; and refined talc, stearate, boric acid powder, polyethylene glycol and other lubricants and the like. Tablets can also be formulated if necessary as tablets with ordinary coatings, such as sugar-coated tablets, gelatin-coated tablets, enteric coated tablets and film coated tablets, as well as double tablets and multilayered tablets.

When a pill formulation is used, a wide variety of carriers that are known in the field can be used. Examples include glucose, lactose, starch, cacao butter, hardened vegetable oil, kaolin, talc and other excipients; gum arabic powder, traganth powder, gelatin, ethanol and other binders; and laminaran, agar and other disintegrators and the like.

When a suppository formulation is used, a wide variety of carriers that are known in the field can be used. Examples include polyethylene glycol, cacao butter, higher alcohol, esters of higher alcohol, gelatin semi-synthetic glyceride and the like.

Capsules are prepared according to ordinary methods by mixing aripiprazole anhydride crystals with the various carriers described above and packing them in hard gelatin capsules, soft capsules, hydroxypropylmethyl cellulose capsules (HPMC capsules) and the like.

In addition, colorants, preservatives, perfumes, flavorings, sweeteners and the like as well as other drugs may be included in the medicinal composition.

In case of forming the pharmaceutical solid oral preparation in the form of granules, it can be prepared by wet granulating a mixed powder of granulating ingredients comprising, aripiprazole anhydride crystals (conventional aripiprazole anhydride crystals or aripiprazole anhydride crystals selected from the group consisting of aripiprazole anhydride type B, C, D, E, F and G crystals) and various carriers which are heretofore well-known in this field, such as excipients, disintegrators, disintegration inhibitors, humectants, absorption accelerators, adsorbents, lubricants, colorants and the like (for the examples of these agents, those of previously mentioned can be referred to) by adding a liquid (generally, water or an aqueous solution containing binding agents). As for the wet granulation, there are various methods are included, for example, fluidized bed granulation, kneading granulation, extruding granulation, rotating granulation and the like can be mentioned. Among these methods, in case of conducting the fluidized bed granulation, the granulating ingredients containing various carriers are mixed with inlet air, then upon continued fluidizing the granulating ingredients and the liquid is sprayed to conduct granulation. In case of conducting the kneading granulation, the granulating ingredients containing various carriers are mixed by agitation, then upon continued agitating the granulating ingredients, granulation is conducted by adding the liquid. After the granulation, if necessary, the obtained granules are sized to make them to the desired size by use of a suitable sieve or a mill having suitable screen size. The granules thus obtained by such a method are dried again in addition to usual drying being conducted when preparing the granules. As for the drying methods, various methods can be applied, for example, methods by use of a fluidized bed dryer, a fan dryer, a vacuum dryer and the like can be mentioned. Generally, drying methods can be conducted under conventional conditions, for example, in case of using the fluidized bed dryer, drying procedure is conducted with an air flow of 0.5 $m^3$/min to 50 $m^3$/min, an inlet air temperature at 70 to 100°C for 10 min to 1 hour. After dried, the granules are subjected to size, then further dried. In case of using the fluidized bed dryer or fan dryer or the like, the drying procedure is conducted under the conditions with an air flow of 0.5 $m^3$/min to 50 $m^3$/min, an inlet air temperature at 70 to 100°C for 1 to 6 hours. In case of using the vacuum dryer, the drying procedure is conducted under the conditions of reduced pressure of about at 0-10 torr of degree of vacuum at 70 to 100°C of jacket temperature for 1 to 6 hour.

The thus prepared granules may be used as they are for the pharmaceucal solid oral preparations, or if necessary, they may be shaped in the form of tablets. Further, the dried granules dried by usual manner are shaped in the form of tablets, then they may be dried again.

The thus prepared pharmaceutical solid oral preparation comprising aripiprazole anhydride crystals hardly changes to hydrates even if they are stored for a long period of time, therefore the pharmaceutical solid oral preparation, of which dissolution rate does not hardly lowered (dissolution rate to maintain maximum drug concentration (Cmax): 60% or higher dissolution rate obtained after 30 minutes at pH 4.5, 70% or higher dissolution rate obtained after 60 minutes at pH 4.5, or 55% or higher dissolution rate obtained after 60 minutes at pH 5.0) can be provided.

Another pharamceutical solid oral preparation can be provided by granulating a conventional aripiprazole hydrate crystals by a method similar to that of mentioned above, and dried by usual manner under similar conditions, then dried again. Alternatively, the dried granules dried by usual manner are shaped to tablets form, then they are dried again, then pharmaceutical solid oral preparations of which dissolution rate does not lowered (dissolution rate to maintain maximum drug concentration (Cmax): 60% or higher dissolution rate obtained after 30 minutes at pH 4.5, 70% or higher dissolution rate obtained after 60 minutes at pH 4.5 or 55% or higher dissolution rate obtained after 60 minutes at pH 5.0) can be provided. These facts can be understood that, the conventional aripiprazole anhydride crystals or the aripiprazole hydrate crystals contained in the pharmaceutical solid oral preparation are changed to "B type crystals" of aripiprazole anhydride by drying twice.

The amount of Aripiprazole Anhydride Crystals B, C, D, E, F and G that should be included in the medicinal composition of the present invention can be selected from a wide range suitable for the indication sought to be treated. Generally, the Aripiprazole Anhydride Crystals B should be present in about 1-70% by weight or particularly about 1-30% by weight based on the medicinal composition.

The method of administration of the medicinal composition of the present invention may be adjusted to suit, for example, the formulation of the drug product, the age, gender and other conditions (including the severity thereof) of the patient. In the case of tablets, pills, liquids, suspensions, emulsions, granules and capsules, for example, administration is oral. In the case of an injection, it is administered intravenously either by itself or mixed with an ordinary replenisher such as glucose or amino acids, or may also be administered by itself intramuscularly, intracutaneously, subcutaneously or intraperitoneally, as necessary. In the case of a suppository, administration is intrarectal.

The dosage of the medicinal composition of the present invention is selected depending on the usage, the age, gender and other conditions of the patient, the severity of the condition and so forth, but ordinarily the amount of aripiprazole anhydride crystals can be about 0.1-10 mg per 1 kg of body weight per day. The preparation which is the unit of administration should contain in the range of about 1-100 mg of Aripiprazole Anhydride Crystals B, more particularly 1-30 mg per unit dose.

The medicinal composition of the present invention is extremely stable, with substantially no decrease in solubility even when stored for long periods of time.

The medicinal composition of the present invention is effective in the prevention and treatment of central nervous system disorders such as schizophrenia and may also be effective in the treatment of intractable (drug-resistant, chronic) schizophrenia with cognitive impairment and intractable (drug-resistant, chronic) schizophrenia without cognitive impairment, anxiety including mild anxiety, mania including bipolar disorder acute mania and acute mania, bipolar disorder, depression including bipolar disorder depression, autism, Down's syndrome, attention deficit hyperactivity disorder (ADHD), Alzheimer's disease, Parkinson's disease and other neurodegenerative diseases, panic, obsessive compulsive disorder (OCD), sleep disorders, sexual dysfunction, alcohol and drug dependency, vomiting, motion sickness, obesity, miparticlee headache and cognitive impairment.

Analytical Methods

(1) The $^1$H-NMR spectrum was measured in DMSO-$d_6$ using TMS as the standard.

(2) Powder X-ray Diffraction

Using a Rigaku Denki RAD-2B diffraction meter, the powder x-ray diffraction pattern was measured at room temperature using a Cu Ka filled tube (35 kV 20mA) as the x-ray source with a wide-angle goniometer, a 1° scattering slit, an 0.15 mm light-intercepting slit, a graphite secondary monochromator and a scintillation counter. Data collection was done in $2\theta$ continuous scan mode at a scan speed of 5°/minute in scan steps of 0.02° in the range of 3° to 40°.

(3) The IR spectrum was measured by the KBr method.

(4) Thermogravimetric/Differential Thermal Analysis

Thermogravimetric/differential thermal analysis was performed using a Seiko SSC 5200 control unit and a TG/DTA 220 simultaneous differential thermal/thermogravimetric measurement unit. 5-10 mg samples were placed in open aluminum pans and heated from 20°C to 200°C in a dry nitrogen atmosphere at a heating rate of 5°C/minute. α-alumina was used as the standard substance.

(5) Differential Scanning Calorimetry

Thermogravimetric/differential thermal analysis was performed using a Seiko SSC 5200 control unit and a DSC 220C differential scanning calorimeter. 5-10 mg samples were placed in crimped aluminum pans and heated from 20°C to 200°C in a dry nitrogen atmosphere at a heating rate of 5°C/minute. α-alumina was used as the standard substance.

(6) Particle Size Measurement

0.1 g of the particles to be measured were suspended in a 20 ml n-hexane solution of 0.5 g soy lecithin, and particle size was measured using a size distribution meter (Microtrack HRA, Microtrack Co.).

(7) Hygroscopicity Test Method

One g of the sample was accurately weighed in a weighing bottle (diameter 5 cm), covered with kimwipes and left to rest in a 60°C/100% RH environment (water/dessicator). 24 hours later, the weighing bottle was removed, transferred to an environment of a room temperature and about 30% RH (magnesium chloride hexahydrate saturated water solution/dessicator) and left to rest for 24 hours and the water content of the sample was measured by the Karl Fischer method.

(8) Solid $^{13}$C-NMR Spectrometry

Solid $^{13}$C-NMR spectrum was measured under the conditions as follows.

Measuring apparatus: CMX-360 Solid State NMR Spectrometer (manufactured by Chemagnetic Inc.)
Computer: SPARC Station 2 (manufactured by SUN Microsystem, Inc.)
OS, Software: Solalis 1.1.1 Rev. B (Registered trademark: UNIX), Spinsight Ver. 2.5
Name of measured pulse: TOSS method (TOSS is a program name of the apparatus) among CP/MAS method.
Width of measured puls: 90° puls was used under the condition of CP.
Measuring sample tube: Test tube made of zirconia, having the outside diameter of 7.5 mm, and inside capacity of 0.8 ml
Revolution: 4250 Hz (Revolution per second Contact time: 1 msec.
Waiting time: 20 sec.
Integrated times: 512 times
Measuring temperature: About 25°C temperature of outside of test tube)
External standard: Methyl group (δ 17.3) of hexamethylbenzene was used as the external standard.

The present invention is explained in more detail below using reference examples, examples, sample preparations and formulation examples.

Reference Example 1

19.4 g of 7-(4-chlorobutoxy)-3,4-dihydrocarbostyril and 16.2 g of 1-(2,3-dichlorophenyl) piperadine 1 hydrochloride were added to 8.39 g of potassium carbonate dissolved in 140 ml of water, and circulated for 3 hours under agitation. After reaction the mixture was cooled and the precipitated crystals filtered out. These crystals were dissolved in 350 ml of ethyl acetate, and about 210 ml of water/ethyl acetate azeotrope removed under reflux. The remaining solution was cooled, and the precipitated crystals filtered out. The resulting crystals were dried for 14 hours at 60°C to produce 20.4 g (74.2%) of raw aripiprazole.
30 g of the raw aripiprazole obtained above was recrystallized from 450 ml of ethanol according to the methods described in Japanese Unexamined Patent Publication No. 191256/1990, and the resulting crystals dried for 40 hours at 80°C to obtain aripiprazole anhydride crystals. The yield was 29.4 g (98.0%).
The melting point (mp) of these aripiprazole anhydride crystals was 140°C, matching the melting point of the aripiprazole anhydride crystals described in Japanese Unexamined Patent Publication No. 191256/1990.
When these crystals were left for 24 hours in a dessicator set at humidity 100%, temperature 60°C, they exhibited hygroscopicity of 3.28% (see Table 1 below).

Reference Example 2

6930 g of the intermediate raw aripiprazole obtained in Reference Example 1 was heat dissolved in 138 liters of hydrous ethanol (water content 20%) according to the method presented at the 4th Japanese-Korean Symposium on Separation Technology, gradually (2-3 hours) cooled to room temperature, and then chilled to near 0°C. The precipitated crystals were filtered out, producing about 7200 g of aripiprazole hydrate (wet state).
The wet-state aripiprazole hydrate crystals obtained above were dried for 30 hours at 80°C to obtain 6480 g (93.5%) of conventional aripiprazole anhydride crystals. The melting point (mp) of these crystals was 139.5°C. These crystals were confirmed by the Karl Fischer method to be anhydrous, with a moisture value of 0.03%.

When left for 24 hours in a dessicator set at humidity 100%, temperature 60°C, these crystals exhibited hygroscopicity of 1.78% (see Table 1 below).

Reference Example 3

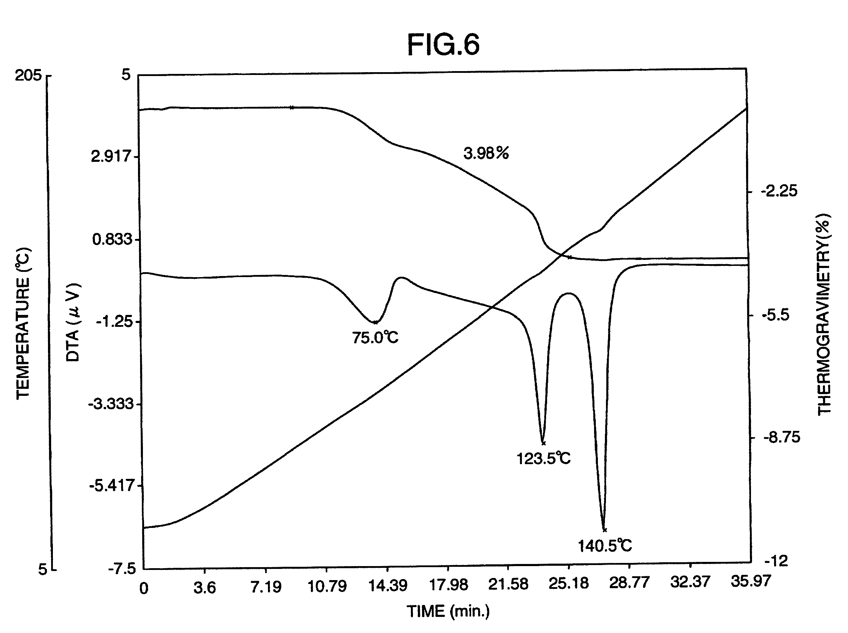

820 g of the intermediate wet-state aripiprazole hydrate obtained in Reference Example 2 was dried for 2 hours at 50°C to obtain 780 g of aripiprazole hydrate crystals. These crystals had a moisture value of 3.82% according to the Karl Fischer method. As shown in Figure 6, thermogravimetric/ differential thermal analysis revealed endothermic peaks at 75.0, 123.5 and 140.5°C. Because dehydration began near 70°C, there was no clear melting point (mp).

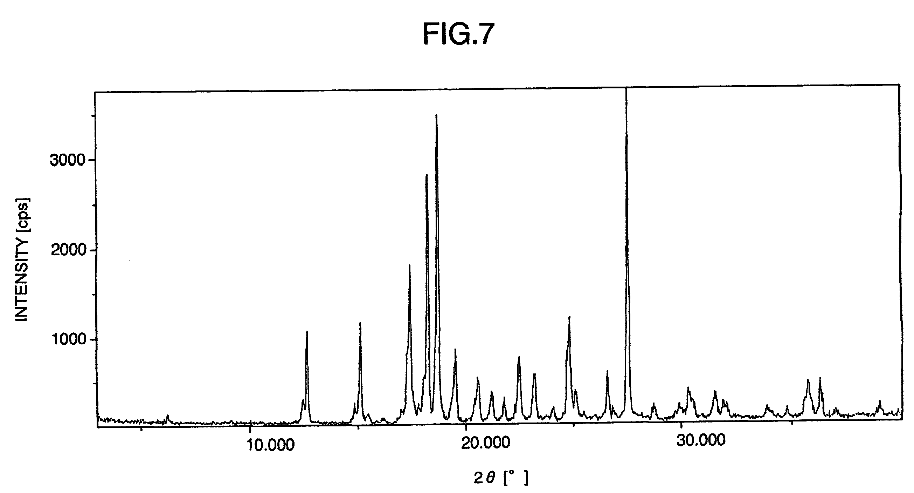

As shown in Figure 7, the powder x-ray diffraction spectrum of aripiprazole hydrate obtained by this method exhibited characteristic peaks at $2\theta$ = 12.6°, 15.1°, 17.4°, 18.2°, 18.7°, 24.8° and 27.5°.

The powder x-ray diffraction spectrum of this aripiprazole hydrate was identical to the powder x-ray diffraction spectrum of aripiprazole hydrate presented at the 4th Joint Japanese-Korean Symposium on Isolation Technology.

Reference Example 4

Preparation of 15 mg tablets containing type I crystals of aripiprazole anhydride obtained in Reference Example 2.

Type-I crystals of aripiprazole anhydride (525 g), lactose (1,995 g), corn starch (350 g) and crystalline cellulose (350 g) were charged in a fluidized bed granulating dryer (Flow coater FLO-5, manufactured by FREUND INDUSTRIAL CO., LTD.), and these granulating ingredients were mixed by fluidizing for about 3 minutes with an inlet air temperature at 70°C and air flow rate of 3 m$^3$/min. Further, the granulating ingredients were upon continued fluidizing under the same condition and sprayed about 1,400 g of the aqueous solution to obtained wet granules. The wet granules were dried under inlet air at temperature at 80°C, for about 15 minutes. The obtained dried granules contained 4.3% of water. (Yield: 99%). The dried granules were subjected to sizing by passing to a sieve of 710 $\mu$m.

About 1% by weight of magnesium stearate was added to the sized granules and mixed, then the granules were supplied to a tablet machine (Rotary single tablet press 12HUK: manufactured by KIKUSUI SEISAKUSHO CO., LTD.), there were obtained tablets, each having 95 mg of weight.

Water content of the tablets was measured according to volumetric titration method (Karl-Fischer method) described in water content measuring method in Japanese Pharmacopoea or the electrical quantity titration method.

Water content measuring method:

Sample (0.1 to 0.5 g) (in case of a tablet, 1 tablet was used) was weighed precisely, and the water content was measured by use of a water content measuring equipment.

Volumetric titration:

Automated water content measuring equipment

Model: KF-06 (manufacture by MITSUBISHI CHEMICAL CORP.)

Electrical quantity titration method:

Automated micro-water content measuring equipment

Model: AQ-7F (manufactured by HIRANUMA SANGYO CO., LTD.)
Automated water vaporization equipment Model: LE-20S (manufactured by HIRANUMA SANGYO CO., LTD.)
Heating temperature: 165±10°C
Nitrogen gas flow rate: about 150 ml/min.

Reference Example 5

Preparation of 15 mg tablets containing type B crystals of aripiprazole anhydride

Type B crystals of aripipraole anhydride (4,500 g), lactose (17,100 g), corn starch (3,000 g) and crystalline cellulose (3,000 g) were charged in a fluidized bed granulating dryer (NEW-MARUMERIZER Model: NQ-500, manufactured by FUJI PAUDAL CO., LTD.), and these granulating ingredients were mixed by fluidizing for about 3 minutes with an inlet air temperature at 70°C, air flow rate of 10 to 15 m$^3$/min. Further, the granulating ingredients were upon continued fluidizing under the same condition, and sprayed about 12,000 g of 5% aqueous solution of hydroxypropyl celulose to obtained wet granules. The wet granules were dried under inlet air at temperature at 85°C, for about 28 minutes. The thus obtained dried granules contained 3.8% of water (measured by the method according to Reference Example 4). (Yield: 96%). The dried granules were subjected to sizing by passing to a sieve of 850 $\mu$m.

About 1% by weight of magnesium stearate was added to the sized granules and mixed, then the granules were supplied to a tablet machine (Rotary single tablet press 12HUK: manufactured by KIKUSUI SEISAKUSHO CO., LTD.), there were obtained tablets, each having 95 mg of weight.

Example 1

500.3 g of the aripiprazole hydrate crystals obtained in Reference Example 3 were milled using a sample mill (small atomizer). The main axis rotation rate was set to 12,000 rpm and the feed rotation rate to 17 rpm, and a 1.0 mm herringbone screen was used. Milling was completed in 3 minutes, resulting in 474.6 g (94.9%) of Aripiprazole Hydrate A powder.

The Aripiprazole Hydrate A (powder) obtained in this way had a mean particle size of 20-25 $\mu$m. The melting point (mp) was undetermined because dehydration was observed beginning near 70°C.

The Aripiprazole Hydrate A (powder) obtained above exhibited an $^1$H-NMR (DMSO-d$_6$, TMS) spectrum which was substantially the same as the $^1$H-NMR spectrum shown in Figure 2. Specifically, it had characteristic peaks at 1.55-1.63 ppm (m, 2H), 1.68-1.78 ppm (m, 2H), 2.35-2.46 ppm (m, 4H), 2.48-2.56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7.17 ppm (m, 1H), 7.28-7.32 ppm (m, 2H) and 10.00 ppm (s, 1H).

The Aripiprazole Hydrate A (powder) obtained above had a powder x-ray diffraction spectrum which was substantially the same as the powder x-ray diffraction spectrum shown in Figure 3. Specifically, it had characteristic peaks at 2$\theta$ = 12.6°, 15.4°, 17.3°, 18.0°, 18.6°, 22.5° and 24.8°. This pattern is different from the powder x-ray spectrum of unmilled aripiprazole hydrate shown in Figure 7.

The Aripiprazole Hydrate A (powder) obtained above had infrared absorption bands at 2951, 2822, 1692, 1577, 1447, 1378, 1187, 963 and 784 cm$^{-1}$ on the IR (KBr) spectrum.

As shown in Figure 1, the Aripiprazole Hydrate A (powder) obtained above had a weak peak at 71.3°C in thermogravimetric/differential thermal analysis and a broad endothermic peak (weight loss observed corresponding to one water molecule) between 60-120°C--clearly different from the endothermic curve of unmilled aripiprazole hydrate (see Figure 6).

Example 2

450 g of the Aripiprazole Hydrate A (powder) obtained in Example 1 was dried for 24 hours at 100°C using a hot air dryer to produce 427 g (yield 98.7%) of Aripiprazole Anhydride Crystals B.

These Aripiprazole Anhydride Crystals B had a melting point (mp) of 139.7°C.

The Aripiprazole Anhydride Crystals B obtained above had an $^1$H-NMR spectrum (DMSO-d$_6$, TMS) which was substantially the same as the $^1$H-NMR spectrum shown in Figure 4. Specifically, they had characteristic peaks at 1.55-1.63 ppm (m, 2H), 1.68-1.78 ppm (m, 2H), 2.35-2.46 ppm (m, 4H), 2.48-2.56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7.17 ppm (m, 1H), 7.28-7.32 ppm (m, 2H) and 10.00 ppm (s, 1H).

The Aripiprazole Anhydride Crystals B obtained above had a powder x-ray diffraction spectrum which was substantially the same as the powder x-ray diffraction spectrum shown in Figure 5. Specifically, they had characteristic peaks at 2$\theta$ = 11.0°, 16.6°, 19.3°, 20.3° and 22.1°.

The Aripiprazole Anhydride Crystals B obtained above had remarkable infrared absorption bands at 2945, 2812, 1678, 1627, 1448, 1377, 1173, 960 and 779 cm$^{-1}$ on the IR (KBr) spectrum.

The Aripiprazole Anhydride Crystals B obtained above exhibited an endothermic peak near about 141.5°C in thermogravimetric/differential thermal analysis.

The Aripiprazole Anhydride Crystals B obtained above exhibited an endothermic peak near about 140.7°C in differential scanning calorimetry.

Even when the Aripiprazole Anhydride Crystals B obtained above were left for 24 hours in a dessicator set at humidity 100%, temperature 60°C, they did not exhibit hygroscopicity exceeding 0.4% (See Table 1 below).

### Example 3

44.29 kg of the Aripiprazole Hydrate A (powder) obtained in Example 1 was dry heated for 18 hours in a 100°C hot air dryer and then heated for 3 hours at 120°C to produce 42.46 kg (yield 99.3%) of Aripiprazole Anhydride Crystals B.

The physicochemical properties of the resulting Aripiprazole Anhydride Crystals B were the same as the physicochemical properties of the Aripiprazole Anhydride Crystals B obtained in Example 2.

The Aripiprazole Anhydride Crystals B obtained in this way did not exhibit hygroscopicity of more than 0.4% even when left for 24 hours in a dessicator set at humidity 100%, temperature 60°C (see Table 1 below).

### Example 4

40.67 kg of the Aripiprazole Hydrate A (powder) obtained in Example 1 was dry heated for 18 hours in a 100°C hot air dryer and then heated for 3 hours at 120°C to produce 38.95 kg (yield 99.6%) of Aripiprazole Anhydride Crystals B.

The physicochemical properties of the resulting Aripiprazole Anhydride Crystals B were the same as the physicochemical properties of the Aripiprazole Anhydride Crystals B obtained in Example 2.

The Aripiprazole Anhydride Crystals B obtained in this way did not exhibit hygroscopicity of more than 0.4% even when left for 24 hours in a dessicator set at humidity 100%, temperature 60°C (see Table 1 below).

Examples 5-10 are useful for injectable or oral solution formulations but not solid dose formulations since they were made by heating Conventional Anhydride or Conventional Hydrate instead of Hydrate A.

### Example 5

The hygroscopic aripiprazole anhydride crystals obtained in Reference Example 1 were heated for 50 hours at 100°C using the same methods as in Example 2. The physicochemical properties of the resulting Aripiprazole Anhydride Crystals B were the same as the physicochemical properties of the Aripiprazole Anhydride Crystals B obtained in Example 2.

The Aripiprazole Anhydride Crystals B obtained in this way did not exhibit hygroscopicity of more than 0.4% even when left for 24 hours in a dessicator set at humidity 100%, temperature 60°C (see Table 1 below).

### Example 6

The hygroscopic aripiprazole anhydride crystals obtained in Reference Example 1 were heated for 3 hours at 120°C using the same methods as in Example 2. The physicochemical properties of the resulting Aripiprazole Anhydride Crystals B were the same as the physicochemical properties of the Aripiprazole Anhydride Crystals B obtained in Example 2.

The Aripiprazole Anhydride Crystals B obtained in this way did not exhibit hygroscopicity of more than 0.4% even when left for 24 hours in a dessicator set at humidity 100%, temperature 60°C (see Table 1 below).

### Example 7

The hygroscopic aripiprazole anhydride crystals obtained in Reference Example 2 were heated for 50 hours at 100°C using the same methods as in Example 2. The physicochemical properties of the resulting Aripiprazole Anhydride Crystals B were the same as the physicochemical properties of the Aripiprazole Anhydride Crystals B obtained in Example 2.

The Aripiprazole Anhydride Crystals B obtained in this way did not exhibit hygroscopicity of more than 0.4% even when left for 24 hours in a dessicator set at humidity 100%, temperature 60°C (see Table 1 below).

### Example 8

The hygroscopic aripiprazole anhydride crystals obtained in Reference Example 2 were heated for 3 hours at 120°C using the same methods as in Example 2. The physicochemical properties of the resulting Aripiprazole Anhydride Crystals B were the same as the physicochemical properties of the Aripiprazole Anhydride Crystals B obtained in Example 2.

The Aripiprazole Anhydride Crystals B obtained in this way did not exhibit hygroscopicity of more than 0.4% even when left for 24 hours in a dessicator set at humidity 100%, temperature 60°C (see Table 1 below).

Example 9

The aripiprazole hydrate crystals obtained in Reference Example 3 were heated for 50 hours at 100°C using the same methods as in Example 2. The physicochemical properties of the resulting Aripiprazole Anhydride Crystals B were the same as the physicochemical properties of the Aripiprazole Anhydride Crystals B obtained in Example 2.
The Aripiprazole Anhydride Crystals B obtained in this way did not exhibit hygroscopicity of more than 0.4% even when left for 24 hours in a dessicator set at humidity 100%, temperature 60°C (see Table 1 below).

Example 10

The aripiprazole hydrate crystals obtained in Reference Example 3 were heated for 3 hours at 120°C using the same methods as in Example 2. The physicochemical properties of the resulting Aripiprazole Anhydride Crystals B were the same as the physicochemical properties of the Aripiprazole Anhydride Crystals B obtained in Example 2.
The Aripiprazole Anhydride Crystals B obtained in this way exhibited hygroscopicity of no more than 0.4% even when left for 24 hours in a dessicator set at humidity 100%, temperature 60°C (see Table 1 below).

Example 11 (Preparation of type C crystals of aripiprazole anhydride)

100 Miligrams of type-I crystals of aripiprazole anhydride obtained in Reference Example 2 were heated about 145°C ($\pm$3°C). In this occasion, there was observed the phenomena that the crystals were once melted, then again crystallized. After that, 100 mg (yield: 100%) of Type C crystals of aripiprazole anhydride were obtained. The melting point of the crystals was 150°C. The crystals were colorless prism form.
The type C crystals of aripiprazole anhydride obtained above had an endothermic curve which was substantially identical to the endothermic curve of thermogravimetric/differential thermal analysis (heating rate: 5°C/minute) shown in Figure 8. Specifically, it showed the endothermic curve around 150.2°C.
The type C crystals of aripiprazole anhydride thus obtained exhibited an $^1$H-NMR spectrum (DMSO-d$_6$, TMS) which was substantially identical to the $^1$H-NMR spectrum (DMSO-d$_6$, TMS) shown in Figure 9. Specifically, it had the characteristic peaks at 1.55-1.63 ppm (m, 2H), 1.68-1.78 ppm (m, 2H), 2.35-2.46 ppm (m, 4H), 2.48-2.56 ppm (m, 4H+DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 7.04 ppm (d, J=8.1 Hz, 1H), 7.11-7.17 ppm (m, 1H), 7.28-7.32 ppm (m, 2H), and 10.00 ppm (s, 1H).
The type C crystals of aripiprazole anhydride obtained above had a powder X-ray diffraction spectrum which was substantially identical to the powder X-ray diffraction spectrum shown in Figure 10. Specifically, it had the characteristic peaks at $2\theta$ = 12.6°, 13.7°, 15.4°, 18.1°, 19.0°, 20.6°, 23.5° and 26.4°.
The type C crystals of aripiprazole anhydride obtained above had an IR spectrum which was substantially identical to the IR (KBr) spectrum shown in Figure 11. Specifically, it had the characteristic infrared absorption bands at 2939, 2804, 1680, 1375 and 780 cm$^{-1}$.
The type C crystals of aripiprazole anhydride obtained above exhibited a solid $^{13}$C-NMR spectrum, which was substantially identical to the solid $^{13}$C-NMR spectrum shown in Figure 12. Specifically, it had the characteristic peaks at 32.8 ppm, 60.8 ppm, 74.9 ppm, 104.9 ppm, 152.2 ppm, 159.9 ppm and 175.2 ppm.
According to the above-mentioned data on endothermic curve of thermogravimetric/differential thermal analysis (heating rate: 5°C/minute) and powder X-ray diffraction spectrum, the formation of the type C crystals of aripiprazole anhydride was confirmed.
When the type C crystals of aripiprazole anhydride crystals obtained above were left for 24 hours in a dessicator where the conditions were set at humidity 100%, and temperature 60°C, then the crystals did not exhibit hygroscopicity higher than 0.4% (see, Table 1 below).

Example 12 (Preparation of type D crystals of aripiprazole anhydride)

The type-I crystals of aripiprazole anhydride obtained in Reference Example 2 were added in 200 ml of toluene, and dissolved by heating at 74°C. After confirmed that it was dissolved completely, the toluene solution was cooled to 7°C, and the precipitated crystals were collected by filtration. The crystals were subjected to air-drying as they were so as to obtain 17.9 g (yield: 89.5%) of type D crystals of aripiprazole anhydride.
The type D crystals of aripiprazole anhydride obtained above had an endothermic curve substantially identical to the endothermic curve of thermogravimetric/differential thermal analysis (heating rate: 5°C/minute) shown in Figure 13. Specifically, it had the endothermic peaks at about 136.8°C and about 141.6°.
The type D crystals of aripiprazole anhydride obtained above exhibited $^1$H-NMR spectrum (DMSO-d$_6$, TMS) which was substantially identical to the $^1$H-NMR spectrum (DMSO-d$_6$, TMS) shown in Figure 14. Specifically, they had the characteristic peaks at 1.55-1.63 ppm (m, 2H), 1.68-1.78 ppm (m, 2H), 2.35-2.46 ppm (m, 4H), 2.48-2.56 ppm (m, 4H+DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 7.04 ppm (d, J=8.1 Hz, 1H), 7.11-7.17 ppm (m, 1H), 7.28-7.32 ppm (m, 2H), and 10.00 ppm (s, 1H).

The type D crystals of aripiprazole anhydride obtained above had a powder X-ray diffraction spectrum which was substantially identical to the powder X-ray diffraction spectrum shown in Figure 15. Specifically, it had the characteristic peaks at $2\theta$ = 8.7°, 11.6°, 16.3°, 17.7°, 18.6°, 20.3°, 23.4° and 25.0°.

The type D crystals of aripiprazole anhydride obtained above had an IR spectrum which was substantially identical to the IR (KBr) spectrum shown in Figure 16. Specifically, it had the characteristic infrared absorption bands at 2946, 1681, 1375, 1273, 1175 and 862 cm$^{-1}$.

The type D crystals of aripiprazole anhydride obtained above exhibited a solid $^{13}$C-NMR spectrum which was substantially identical to the solid $^{13}$C-NMR spectrum shown in Figure 17. Specifically, it had the characteristic peaks at 32.1 ppm, 62.2 ppm, 66.6 ppm, 104.1 ppm, 152.4 ppm, 158.5 ppm and 174.1 ppm.

According to the above-mentioned data on the endothermic curve of thermogravimetric/differential thermal analysis (heating rate: 5°C/minute) and powder X-ray diffraction spectrum, the formation of type D crystals of aripiprazole anhydride was confirmed.

When the type D crystals of aripiprazole anhydride crystals obtained above were left for 24 hours in a dessicator where the conditions were set at humidity 100%, and temperature 60°C, the crystals did not have hygroscopicity higher than 0.4% (see, Table 1 below).

Example 13 (Preparation of type D crystals of aripiprazole anhydride)

1,200 Grams of the type-I crystals of aripiprazole anhydride obtained in Reference Example 2 were dissolved in 18 liters of toluene, with heating. This toluene solution was cooled to 40°C, and 36 g of type-D crystals of aripiprazole anhydride obtained in Example 12 were added as seed crystals, then the solution was cooled to 10°C and allowed to stand as it is. The precipitated crystals were collected by filtration, dried at 60°C for 18 hours to obtain 1,073 g (yield: 86.8%) of type D crystals of aripiprazole anhyride (purity: 100%). The crystals were colorless plate form.

The type D crystals of aripiprazole anhydride had an endothermic curve substantially identical to the endothermic curve of thermogravimetric/differential thermal analysis (heating rate: 5°C/minute) shown in Figure 13. Specifically, it had the endothermic peaks around about 136.8°C and about 141.6°.

The type D crystals of aripiprazole anhydride obtained above exhibited an $^1$H-NMR spectrum (DMSO-d$_6$, TMS) which was substantially identical to the $^1$H-NMR spectrum (DMSO-d$_6$, TMS) shown in Figure 14. Specifically, it had the characteristic peaks at 1.55-1.63 ppm (m, 2H), 1.68-1.78 ppm (m, 2H), 2.35-2.46 ppm (m, 4H), 2.48-2.56 ppm (m, 4H+DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 7.04 ppm (d, J=8.1 Hz, 1H), 7.11-7.17 ppm (m, 1H), 7.28-7.32 ppm (m, 2H), and 10.00 ppm (s, 1H).

The type D crystals of aripiprazole anhydride obtained above had a powder X-ray diffraction spectrum which was substantially identical to the powder X-ray diffraction spectrum shown in Figure 15. Specifically, it had the characteristic peaks at $2\theta$ = 8.7°, 11.6°, 16.3°, 17.7°, 18.6°, 20.3°, 23.4° and 25.0°.

The type D crystals of aripiprazole anhydride obtained above had an IR spectrum which was substantially identical to the IR (KBr) spectrum shown in Figure 16. Specifically, it had characteristic infrared absorption bands at 2946, 1681, 1375, 1273, 1175 and 862 cm$^{-1}$.

The type D crystals of aripiprazole anhydride obtained above had a solid $^{13}$C-NMR spectrum which was substantially identical to the solid $^{13}$C-NMR spectrum shown in Figure 17. Specifically, it had the characteristic peaks at 32.1 ppm, 62.2 ppm, 66.6 ppm, 104.1 ppm, 152.4 ppm, 158.5 ppm and 174.1 ppm.

According to the above-mentioned data on the endothermic curve of thermogravimetric/differential thermal analysis (heating rate: 5°C/minute) and powder X-ray diffraction spectrum, the formation of type D crystals of aripiprazole anhydride was confirmed.

When the type D crystals of aripiprazole anhydride crystals obtained above were left for 24 hours in a dessicator where the conditions were set at humidity 100%, and temperature 60°C, the crystals did not exhibit hygroscopicity higher than 0.4% (see, Table 1 below).

Example 14 (Preparation of type E crystals of aripiprazole anhydride)

40 Grams of type-I crystals of aripiprazole anhydride obtained in Reference Example 2 was dissolved in 1000 ml of acetonitrile with heating at 80°C. This acetonitrile solution was cooled to about 70°C by taking for about 10 minutes, and was kept at this temperature for about 30 minutes to precipitate the seed crystals. Next, the temperature of said solution was slowly risen to 75°C, and the crystals were grown up by keeping this temperature for 1 hour. Then, the solution was cooled to 10°C by taking about 4 hours, and the precipitated crystals were collected by filtration. Thus obtained crystals were subjected to air-drying overnight, there were obtained 37.28 g (yield: 93.2%) of type E crystals of aripiprazole anhydride (purity: 100%). The melting point of these crystals was 145°C, and the crystals were colorless needle form.

The type E crystals of aripiprazole anhydride had an endothermic curve substantially identical to the endothermic curve of thermogravimetric/differential thermal analysis (heating rate: 5°C/minute) shown in Figure 18. Specifically, it had endothermic peak at about 146.5°.

The type E crystals of aripiprazole anhydride obtained above exhibited an $^1$H-NMR spectrum (DMSO-$d_6$, TMS) which was substantially identical to the $^1$H-NMR spectrum (DMSO-$d_6$, TMS) shown in Figure 19. Specifically, it had the characteristic peaks at 1.55-1.63 ppm (m, 2H), 1.68-1.78 ppm (m, 2H), 2.35-2.46 ppm (m, 4H), 2.48-2.56 ppm (m, 4H+DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 7.04 ppm (d, J=8.1 Hz, 1H), 7.11-7.17 ppm (m, 1H), 7.28-7.32 ppm (m, 2H), and 10.00 ppm (s, 1H).

The type E crystals of aripiprazole anhydride obtained above had a powder X-ray diffraction spectrum which was substantially identical to the powder X-ray diffraction spectrum shown in Figure 20. Specifically, it had the characteristic peaks at $2\theta$ = 8.0°, 13.7°, 14.6°, 17.6°, 22.5° and 24.0°.

The type E crystals of aripiprazole anhydride obtained above had an IR spectrum which was substantially identical to the IR (KBr) spectrum shown in Figure 21. Specifically, it had the characteristic infrared absorption bands at 2943, 2817, 1686, 1377, 1202, 969 and 774 cm$^{-1}$.

According to the data on the endothermic curve of thermogravimetric/differential thermal analysis (heating rate: 5°C/minute) and powder X-ray diffraction spectrum, the formation of type E crystals of aripiprazole anhydride was confirmed.

When the type E crystals of aripiprazole anhydride obtained above were left for 24 hours in a dessicator where the conditions were set at humidity 100%, and temperature 60°C, the crystals did not exhibit hygroscopicity higher than 0.4% (see, Table 1 below).

Example 15 (Preparation of type F crystals of aripiprazole anhydride)

140 Grams of type-I crystals of aripiprazole anhydride obtained in Reference Example 2 were suspended in 980 ml of acetone and continued to reflux for 7.5 hours with stirring. Next, the suspension was filtered in hot condition, and crystals separated out were subjected to air-drying for 16 hours at room temperature, there was obtained 86.19 g (yield: 61.6%) of type F crystals of aripiprazole anhydride (purity: 100%). The crystals were colorless prism form.

The type F crystals of aripiprazole anhydride had an endothermic curve substantially identical to the endothermic curve of thermogravimetric/differential thermal analysis (heating rate: 5°C/minute) shown in Figure 22. Specifically, it had the exothermic peaks at about 137.5°C and about 149.8°C.

The type F crystals of aripiprazole anhydride obtained above exhibited an $^1$H-NMR spectrum (DMSO-$d_6$, TMS) which was substantially identical to the $^1$H-NMR spectrum (DMSO-$d_6$, TMS) shown in Figure 23. Specifically, it had the characteristic peaks at 1.55-1.63 ppm (m, 2H), 1.68-1.78 ppm (m, 2H), 2.35-2.46 ppm (m, 4H), 2.48-2.56 ppm (m, 4H+DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 7.04 ppm (d, J=8.1 Hz, 1H), 7.11-7.17 ppm (m, 1H), 7.28-7.32 ppm (m, 2H), and 10.00 ppm (s, 1H).

The type F crystals of aripiprazole anhydride obtained above had a powder X-ray diffraction spectrum which was substantially identical to the powder X-ray diffraction spectrum shown in Figure 24. Specifically, it had the characteristic peaks at $2\theta$ = 11.3°, 13.3°, 15.4°, 22.8°, 25.2° and 26.9°.

The type F crystals of aripiprazole anhydride obtained above had an IR spectrum which was substantially identical to the IR (KBr) spectrum shown in Figure 25. Specifically, it had the characteristic infrared absorption bands at 2940, 2815, 1679, 1383, 1273, 1177, 1035, 963 and 790 cm$^{-1}$

According to the data on endothermic curve of thermogravimetric/differential thermal analysis (heating rate: 5°C/minute) and powder X-ray diffraction spectrum, the formation of type F crystals of aripiprazole anhydride was confirmed.

When the type F crystals of aripiprazole anhydride crystals obtained above were left for 24 hours in a dessicator where the conditions were set at humidity 100%, and temperature 60°C, the crystals did not exhibit hygroscopicity higher than 0.4% (see, Table 1 below).

Table 1

| Sample | Initial Moisture Content (%) | Moisture Content After 24 hrs (%) |
|---|---|---|
| Reference Example 1 | 0.04 | 3.28 |

(continued)

| Sample | Initial Moisture Content (%) | Moisture Content After 24 hrs (%) |
|---|---|---|
| Reference Example 2 | 0.04 | 1.78 |
| Example 2 | 0.04 | 0.04 |
| Example 3 | 0.02 | 0.02 |
| Example 4 | 0.02 | 0.02 |
| Example 5 | 0.04 | 0.04 |
| Example 6 | 0.04 | 0.04 |
| Example 7 | 0.04 | 0.03 |
| Example 8 | 0.04 | 0.03 |
| Example 9 | 0.03 | 0.01 |
| Example 10 | 0.05 | 0.05 |
| Example 11 | 0.03 | 0.03 |
| Example 12 | 0.04 | 0.03 |
| Example 13 | 0.04 | 0.03 |
| Example 14 | 0.06 | 0.09 |
| Example 15 | 0.04 | 0.04 |

Example 16

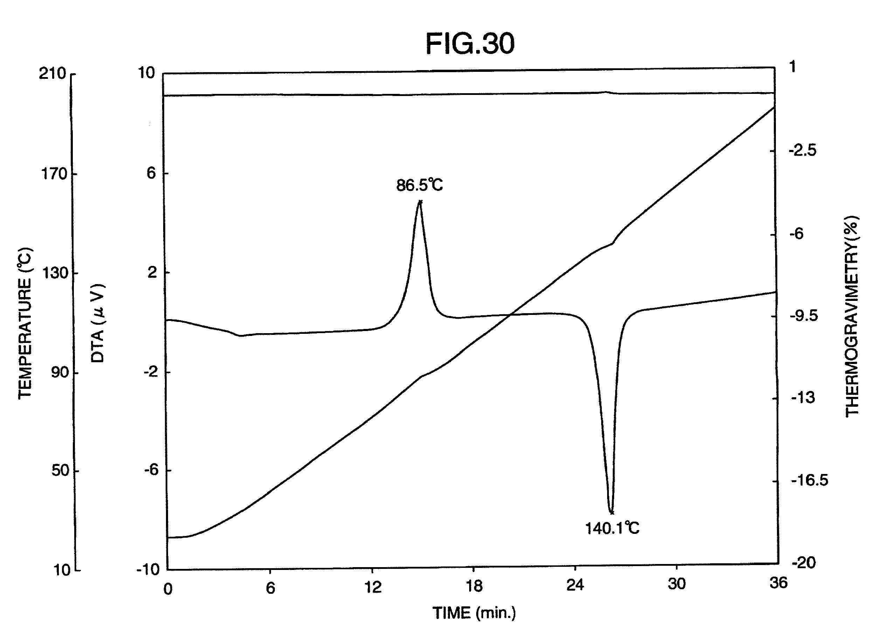

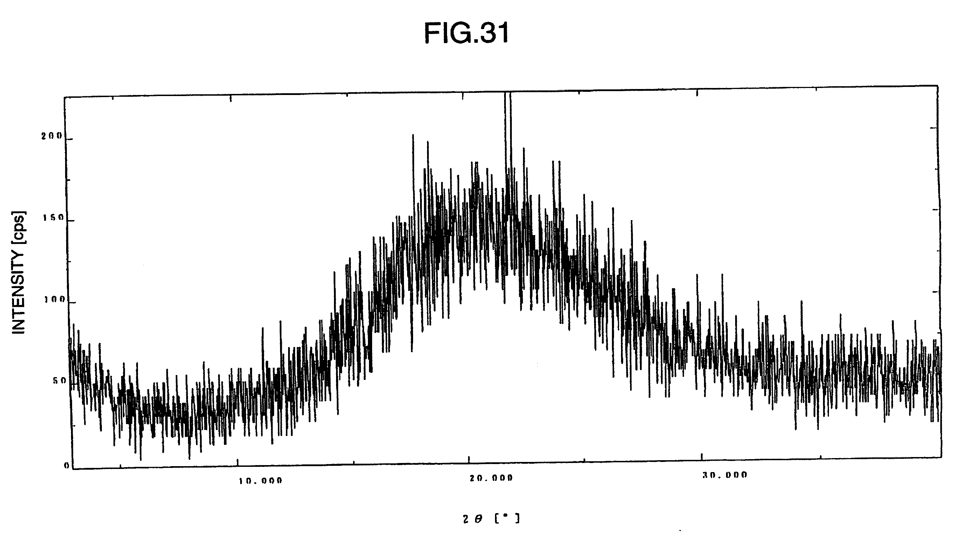

a) Type I crystals of aripiprazole anhydride (10 g) obtained in Reference Example 2 was charged in a stainless steel round tray (diameter: 80 mm), and heated to about 170°C so as to melted completely. When this melted liquid was cooled, then it solidified clarity with pale brawn in color, the solid was peeled off from the stainless steel round tray, there was obtained 9.8 g (yield: 98%) of glassy state of aripiprazole anhydride. The obtained glassy state product was characterized by having no significant peak observed in a powder X-ray determination. (cf. Figure 31).
According to the thermogravimetric/ differential thermal analysis (heating rate: 5°C/ minute), as shown in Figure 30, an exothermic peak of type B crystals of aripiprazole anhydride was observed at around 86.5°C. While, an endothermic peak of type B crystals of aripiprazole anhydride owing to melting was observed at around 140.1°C.
b) When the glassy state of aripiprazole anhydride obtained in Example 16-a) were charged in a sealed vessel and left to stand at room temperature for about 6 months, then type G crystals of aripiprazole anhydride having white in color was obtained by changing the color from pale brown (25 g, yield: 100%). Melting point: 138 to 139°C.

The type G crystals of aripiprazole anhydride had an endothermic curve which was substantially identical to the thermogravimetric/differential thermal analysis (heating rate: 5°C/min.) endothermic curve shown in Figure 26, more particulaly, it has an endothermic peak around 141.0°C and an exothermic peak around 122.7°C.
The type G crystals of aripiprazole anhydride obtained as above exhibited an $^1$H-NMR spectrum which was substantially identical to the $^1$H-NMR spectrum (DMSO-$d_6$, TMS) shown in Figure 27. Specifically, it has characteristic peaks at 1.55 - 1.63 ppm (m, 2H), 1.68 - 1.78 ppm (m, 2H), 2.35 - 2.46 ppm (m, 4H), 2.48 - 2.56 ppm (m, 4H + DMSO), 2.78 ppm (t, J=7.4 Hz, 2H), 2.97 ppm (brt, J=4.6 Hz, 4H), 3.92 ppm (t, J=6.3 Hz, 2H), 6.43 ppm (d, J=2.4 Hz, 1H), 6.49 ppm (dd, J=8.4 Hz, J=2.4 Hz, 1H), 7.04 ppm (d, J=8.1 Hz, 1H), 7.11 - 7.17 ppm (m, 1H), 7.28 - 7.32 ppm (m, 2H) and 10.00 ppm (s, 1H).
The type G crystals of aripiprazole anhydride obtained as above had a powder X-ray diffraction spectrum which was substantially identical to the powder X-ray diffraction spectrum shown in Figure 28. Specifically, it has characteristic peak at 2θ = 10.1°, 12.8°, 15.2°, 17.0°, 17.5°, 19.1°, 20.1°, 21.2°, 22.4°, 23.3°, 24.5° and 25.8°.
The type G crystals of aripiprazole anhydride obtained above had an IR spectrum which was substantially identical to the IR (KBr) spectrum shown in Figure 29. Specifically, it has clear infrared absorption bands at 2942, 2813, 1670, 1625, 1377, 1195, 962 and 787 cm$^{-1}$.

Example 17

a) Preparation of granules of 30 mg tablets containing type B crystals of aripiprazole anhydride for additional drying

Type B crystals of aripiprazole anhydride (1,500 g), lactose (5,700 g), corn starch (1,000 g) and crystalline cellulose (1,000 g) were charged in a fluidized bed granulating dryer (Flow Coater Model FLO-5M; manufactured by FROINT SANGYO KABUSHIKI KAISHA), and these granulating ingredients were mixed by fluidizing for about 3 minutes with an inlet air temperature at 60°C, air flow rate of 3 to 4 $m^3$/min. Further, the granulating ingredients were continued fluidizing under the same condition, and sprayed with about 4,000 g of 5% aqueous solution of hydroxypropyl celulose to obtain wet granules. The wet granules were dried under an inlet air temperature at 85°C, for about 20 minutes. The obtained dried granules contained 3.8% of water (measured by the method according to Reference Example 4).

b) The dried granules (4 kg) obtained in Example 17-a) were sized by use of a mill (FIORE F-0: manufactured by TOKUJU CORPORATION).
The sized granules (3 kg) were charged in a fluidized bed granulating dryer (Flow Coater Model FLO-5M; manufactured by FREUND INDUSTRIAL CO., LTD.), and these granulating ingredients were dried under an inlet air temperature at 85°C, and air flow rate of 2 $m^3$/min for 2 hours. The obtained dried granules contained 3.6% of water (measured by the method according to Reference Example 4).
About 1% by weight of of magnesium stearate was added to the sized granules and mixed, then the granules were supplied to a tabletting machine (a Rotary single tablet press, Model VIRGO: manufactured by KIKUSUI SEISAKUSHO CO., LTD.), and there were obtained tablets, each having 190 mg of weight.
c) The dried granules (3 kg) obtained in Example 17-a) were charged in a vacuum dryer (vacuum granulating dryer model; VG-50: manufactured by KIKUSUI SEISAKUSHO CO., LTD.), and dried at 70°C of a jacket temperature, under a reduced pressure at 5 torr of degree of vacumm for 1 hour. The thus obtained dried granules contained 3.1% of water (measured by the method according to Reference Example 4). The dried granules were subjected to sizing by passing to a sieve of 850 $\mu$m.

About 1% by weight of magnesium stearate was added to the sized granules and mixed, then the granules were supplied to a tablet machine (Rotary single tablet press, Model VIRGO: manufactured by KIKUSUI SEISAKUSHO CO., LTD.), and there were obtained tablets, each having 190 mg of weight.

Example 18

a) Preparation of 30 mg tablets containing type B crystals of aripiprazole anhydride

Aripiprazole anhydride (type B crystals) (4,500 g), lactose (17,100 g), corn starch (3,000 g) and crystalline cellulose (3,000 g) were charged in a fluidized bed granulating dryer (NEW-MARUMERIZER Model: NQ-500, manufactured by FUJI PAUDAL CO., LTD.), and these granulating ingredients were mixed by fluidizing for about 3 minutes with an inlet air temperature at 70°C, air flow rate of 10 - 15 $m^3$/min. Further, the granulating ingredients were continued fluidizing under the same condition, and were sprayed with about 12,000 g of 5% aqueous solution of hydroxypropyl celulose to obtain wet granules. The wet granules were dried under inlet air at temperature of 85°C, for about 30 minutes. The obtained dried granules contained 3.6% of water (measured by the method according to Reference Example 4). (Yield: 96%). The dried granules were sized by passing to a mill (FIOLE F-0: manufactured by TOKUJU CORPORATION).
About 1% by weight of magnesium stearate was added to the sized granules and mixed, then the granules were supplied to a tablet machine (a Rotary single tablet press, VIRGO: manufactured by KIKUSUI SEISAKUSHO CO., LTD.), and there were obtained tablets, each having 190 mg of weight.

b) The tablets (5 kg) obtained in Example 18-a) were charged in a fan dryer (AQUA COATER AQC-48T, manufactured by FREUND INDUSTRIAL CO., LTD.), and dried under inlet air at temperature of 90°C, air flow rate of 2 $m^3$/min for 6 hours. The obtained dried granules contained 3.3% of water (measured by the method according to Reference Example 4).
c) The dreid tablets (3 kg) obtained in Example 18-a) were charged in a vacuum dryer (vacuum granulating dryer, VG-50: manufactured by KIKUSUI SEISAKUSHO CO., LTD.), and dried at 80°C of a jacket temperature, under reduced pressure of 5 torr of degree of vacumm for 4 hours. The obtained dried tablets contained 2.7% of water (measured by the method according to Reference Example 4).

Example 19

a) By the procedures similar to those of Example 18-a), there were obtained tablets (containing type I crystals of aripiprazole anhydride obtained in Reference Example 2), each having 190 mg of weight,
b) The tablets were dried by the procedures similar to those of Example 18-b), except that air inlet temperature was 100°C and dried for 1 hour.
c) The tablets were dried by the procedures similar to those of Example 18-b), except that inlet air temperature was 100°C and dried for 3 hours.

Example 20

By the procedures similar to those of Example 18-a), there were obtained tablets, each having 190 mg of weight, containing type C crystals of aripiprazole anhydride.

Example 21

By the procedures similar to those of Example 18-a), there were obtained tablets, each having 190 mg of weight, containing type D crystals of aripiprazole anhydride.

Example 22

a) Aripiprzole hydrate crystals (156 g) obtained in Reference Example 3, lactose (570 g), corn starch (100 g) and crystalline cellulose (100 g) were charged in a fluidized bed granulating dryer (NEW-MARUMERIZER, NQ-160: manufactured by FUJI POWDAL CO., LTD.), and these granulating ingredients were mixed under fluidizing for about 3 minutes with an inlet air temperature at 60°C, air flow rate of 1.0 to 1.5 m$^3$/min, and rotating disc with rotary speed of 400 rpm. Further, the granulating ingredients were continued fluidizing under the same condition, and sprayed about 500 g of 4% aqueous solution of hydroxypropyl celulose to obtain wet granules. The inlet air temperature was elevated up to 85°C, and dried until the temperature of the product was reatched to 46°C. The obtained dried granules were sized by passing to a sieve of 850 $\mu$m. The dried granules contained 4.37% of water (measured by the method according to Reference Example 4).
b) The dried granules (200 g) obtained in Example 22-a) were charged in a fluidized bed dryer (multiplex, MP-01: manufactured by POWREX CORPORATION), and dried at 85°C of inlet air temperature, air flow rate of 0.5 m$^3$/min for 2 hours. The dried granules contained 3.50% of water (measured by the method according to Reference Example 4).
c) The dried granules (100 g) obtained in Example 22-a) were charged in a vacuum dryer (vacuum granulating dryer LCV-232: manufactured by TABAI CO., LTD.), and dried 80°C of tray temperature, about 760 mmHg of degree of vacuum for 2 hours. The dried granules were further dried similarly for 6 hours. The dried granules contained 3.17% of water (the product being dried for 2 hours: measured by the method according to Reference Example 4). The further dried granules contained 2.88% of water (the product being dried for 6 hours: measured by the method according to Reference Example 4).
d) About 1% by weight of magnesium stearate was added to the sized granules being obtained in Example 22-b) and mixed, then the mixed granules were supplied to a tablet machine (Single type Tablet machine No. 2B: manufactured by KIKUSUI SEISAKUSHO CO., LTD.), and tabletted with punch, there were obtained tablets, each having 191 mg of weight.
e) About 1% by weight of magnesium stearate was added to the sized granules being obtained in Example 22-c) and mixed, then the mixed granules were supplied to a tablet machine (Single type Tablet machine No. 2B: manufactured by KIKUSUI SEISAKUSHO CO., LTD.), and tabletted with punch, there were obtained tablets, each having 191 mg of weight.

Dissolution Test

Each tablets of the pharmaceutical solid oral preparations obtained previously was kept, repectively under the open at 25°C/60% RH for 6 months, and at 40°C/75% RH for 1 week, then their dissolution rates were measured by the following methods. The dissolution rates obtained from 60 minutes after the exposure are shown in Tables 2 and 3. The dissolution rates after 60 minutes, using the tablets kept under the open at 40°C/75% RH for 2 weeks, are shown in Tables 4 and 5. The dissolution rates after 60 minutes, using the tablets kept under the open condition at 40°C/75% RH for 1 week, are shown in Table 6.

Dissolution test equipment: USP
Model: NTR-6100 (manufactured by TOYAMA SANGYO CO., LTD.)
Model: DT-610(manufactured by JASCO CORPORATION)

a) Method of dissolution test of the 15 mg tablet

One tablet (containing 15 mg each of aripiprazole anhydride or hydrate) was tested by using 900 ml of acetic acid buffer solution (pH 5.0) (Note: 1) as the test solution, and by rotating a paddle at 100 rpm according to the method of USP (United States Pharmacopoea) (Note: 2).
The test solutions obtained respectively from 10 minutes, 20 minutes, 30 minutes, 45 minutes and 60 minutes after the start of test are named as T10, T20, T30, T45 and T60.
On the other hand, about 0.05 g of standard sample of aripiprazole was weighed accurately, dissolved in ethanol (95%) so as to make exactly 50 ml of ethanol solution. Twenty (20) ml of this ethanol solution was taken accurately, and to prepared exactly 1000 ml of the standard solution by adding 0.01 mol/ liter of hydrochloric acid reagent solution (Note: 3).
The test solutions and the standard solution were subjected to filtration, respectively by using a filter having micropores of 10 to 20 $\mu$m in diameters, then each of the filtrates were introduced to a spectrophotometer installed with flow cell (cell length: 10 mm), and to measure the absorbance of wave length at 249 nm and absorbance of wave length at 325 nm and determined the differences between absorbances to named as At10, At20, At30, At45, At60 and As, respectively.
After the measurements, the test solutions of T10, T20, T30 and T45 were put back to the test vessels respectively. Further, similar procedures were conducted to other 5 samples of the test solutions.

$$\text{Dissolution rate (\%) relating to the indicated amount of aripiprazole} =$$
$$\text{Amount of the standard sample of aripiprazole (mg)} \times At \times As \times 9/5 \times 20/C$$

wherein,

At: At10, At20, At30, At45 or At60
As: standard solution
C: Indicated amount of aripiprazole (mg)

(Note:1) Water was added to 1.97 g of acetic acid (100) and 9.15 g of sodium acetate-trihydrate to make 1000 ml of solution (0.1 mol/l).
(Note:2) Paddle method
(Note:3) Water was added to 100 ml of 0.1 mol/l hydrochloric acid (Note:4) to make 1000 ml of solution.
(Note:4) Water was added to 0.9 ml of hydrochloric acid to make 1000 ml of solution.

b) Method of dissolution test of the 30 mg tablet

One tablet each of the pharmaceutical solid oral preparations (containing 30 mg each of aripiprazole anhydride or hydrate) was tested by using 900 ml of acetic acid buffer solution (pH 4.5) (Note: 5) as the test solution, and to conduct the test by rotating a paddle at 75 rpm in accordance with the method of USP (United States Pharmacopoea)(Note: 6).
The test solutions obtained respectively from 10 minutes, 20 minutes, 30 minutes 45 minutes and 60 minutes after the start of test, were named as T10, T20, T30, T45 and T60.
On the other hand, about 0.05 g of the standard sample of aripiprazole was weighed accurately, and dissolved in ethanol (95%) so as to made exactly 50 ml of the ethanol solution. Twenty (20) ml of the ethanol solution was taken accurately, and prepared exactly 1000 ml of the standard solution by adding 0.01 mol/liter of hydrochloric acid reagent solution (Note: 7).

The test solutions and standard solution were subjected to filtration, respectively by using a filter having micropores of 10 to 20 $\mu$m in diameters, then each of the filtrates were introduced to a spectrophotometer in which a flow cell (cell length: 10 mm) was installed, and measured the absorbance of wave length at 249 nm and absorbance of wave length at 325 nm, and the difference between these absorbances were named as At10, At20, At30, At45, At60 and As, respectively.

After the measurements, the test solutions of T10, T20, T30 and T45 were put back respectively to the test vessels. Further, similar procedures were conducted to other 5 samples of the test solutions.

$$\text{Dissolution rate (\%) relating to the indicated}$$

$$\text{amount of aripiprazole} =$$

$$\text{Amount of the standard sample of aripiprazole (mg)}$$

$$\times \text{At} \times \text{As} \times 9/5 \times 20/C$$

wherein,

At: At10, At20, At30, At45 or At60

As: standard solution

C: Indicated amount of aripiprazole (mg)

(Note:5) Water was added to 1.91 g of acetic acid (100) and 2.99 g of sodium acetate·trihydrate to made 1000 ml of solution (0.05 mol/l).

(Note:6) Paddle method

(Note:7) Water is added to 100 ml of 0.1 mol/l hydrochloric acid (Note:8) to made 1000 ml of solution.

(Note:8) Water was added to 0.9 ml of hydrochloric acid to make 1000 ml of solution.

Table 2

| Samples used | Open at 25°C/60% RH | | Open at 40°C/75% RH | |
|---|---|---|---|---|
| | Initial | After 6 months | Initial | After 1 week |
| Tablet(15 mg) of Reference Example 4 | 83.4% | 44.3% | 83.4% | 44.1% |
| Tablet(15 mg) of Reference Example 5 | 90.1% | 61.9% | 90.1% | 65.2% |

Table 3

| Samples used | Open at 25°C/60% RH | | Open at 40°C/75% RH | |
|---|---|---|---|---|
| | Initial | After 6 months | Initial | After 1 week |
| Tablet (30 mg) of Example 18-a) | 96.7% | 77.1% | 96.7% | 75.9% |
| Tablet (30 mg) of Example 17-b) | 96.5% | 93.6% | 95.0% | 92.2% |
| Tablet(30 mg) of Example 17-c) | 97.0% | 96.3% | 94.7% | 94.8% |
| Tablet (30 mg) of Reference Example 18-b) | 97.2% | 95.3% | 97.2% | 97.8% |
| Tablet(30 mg) of Reference Example 18-c) | 97.8% | 96.3% | 97.8% | 96.9% |

Table 4

| Samples used | Initial | After 2 weeks |
|---|---|---|
| Samples used Tablet (30 mg) of Example 19-a) | 89.8% | 66.9% |
| Tablet (30 mg) of Example 19-b) | - | 79.8% |
| Tablet (30 mg) of Example 19-c) | - | 85.9% |

Table 5

| Samples used | Initial | After 2 weeks |
|---|---|---|
| Tablet (30 of Example 18-a) | 94.8% | 94.7% |
| Tablet (30 mg) of Example 20 | 93.7% | 93.1% |
| Tablet (30 mg) of Example 21 | 94.8% | 90.9% |

Table 6

| Samples used | Initial | After 1 weeks |
|---|---|---|
| Tablet (30 mg) of Example 22-d) | 96.5% | 84.5% |
| Tablet (30 mg) of Example 22-e) (dreid for 2 hours) | 92.5% | 74.4% |
| Tablet (30 mg) of Example 22-e) (dreid for 6 hours) | 96.2% | 83.4% |
| (Note: Dissolution tests in Table 5 were conducted similarly to the procedures in the above-mentioned "b) Method of dissolution test of the 30 mg tablet" except that by using 900 ml of acetic acid buffer solution (pH 4.0) as the test solution, and by rotating a paddle at 50 rpm. | | |

As can be seen clearly from the data shown in Table 2, in comparison with the 15 mg tablet containing conventional aripiprazole anhydride crystals (Reference Example 4), the 15 mg tablet containing type B crystals of aripiprazole anhydride (Reference Example 5) had the dissolution rate to maintain maximum drug concentration (Cmax), at pH 5.0 after 60 minutes, even though such tablet was kept under the open at 25°C/60%RH for 6 months and under the open at 40°C/75%RH for 1 week.

As can be seen clearly from the data shown in Table 3, even though 30 mg tablets (Examples 17-b) and 17-c)) prepared from twice dried granules of type B crystals of aripiprazole anhydride, and 30 mg tablets (Examples 18-b) and 18-c)) prepared from further dried pharmaceutical solid oral preparation containing type B crystals of aripiprazole anhydride were subjected to keep under the open at 25°C/60%RH for 6 months or 40°C/75%RH for 1 week, the dissolution rates of these tablets obtained 60 minutes after the test at pH 4.5 were not substantially lowered.

As can be seen clearly from the data shown in Table 4, when 30 mg tablets (Examples 19-a), 19-b) and 19-c)) containing conventional aripiprazole anhydride crystals were further dried and subjected to keep under open at 40°C/75%RH for 2 weeks, then the dissolution rates of the tablets obtained 60 minutes after the test at pH 4.5 were the dissolution rates to maintain maximum drug concentration (Cmax).

As can be seen clearly from the data shown in Table 5, when 30 mg tablet (Example 18-a)) containing type B crystals of aripiprazole anhydride, 30 mg tablet (Example 20) containing type C crystals of aripiprazole anhydride and 30 mg tablet (Example 21) containing type D crystals of aripirazole anhydride were subjected to keep under open at 40°C/75%RH for 2 weeks, then the dissolution rates of the tablets obtained 60 minutes after the test at pH 4.0 were not substantially lowered.

As can be seen clearly from the data shown in Table 6, when 30 mg tablets (Examples 22-d) and 22-e)) prepared from granules of conventional aripiprazole hydrate being twice dried, and subjected to keep under open at 40°C/75%RH for 1 week, then the dissolution rates of the tablets obtained 60 minutes after the test at pH 4.5 were the dissolution rates to maintain maximum drug concentration (Cmax).

<u>Sample Preparation 1</u>

| | |
|---|---|
| Aripiprazole anhydride crystals B | 5 mg |
| Starch | 131 mg |
| Magnesium stearate | 4 mg |
| Lactone | 60 mg |
| Total | 200 mg |

Tablets containing the above ingredients in each tablet were prepared by formulation methods known to one skilled in the art of pharmaceutical formulation.

Sample Preparation 2

| | |
|---|---|
| Type C crystals of aripiprazole anhydride | 5 mg |
| Starch | 131 mg |
| Magnesium stearate | 4 mg |
| Lactose | 60 mg |
| Total | 200 mg |

In accordance with an ordinary method, tablet preparation, containing the above-mentioned ingredients per 1 tablet was prepared.

Sample Preparation 3

| | |
|---|---|
| Type D crystals of aripiprazole anhydride | 5 mg |
| Starch | 131 mg |
| Magnesium stearate | 4 mg |
| Lactose | 60 mg |
| Total | 200 mg |

In accordance with an ordinary method, tablet preparation, containing the above-mentioned ingredients per 1 tablet was prepared.

Sample Preparation 4

| | |
|---|---|
| Type E crystals of aripiprazole anhydride | 5 mg |
| Starch | 131 mg |
| Magnesium stearate | 4 mg |
| Lactose | 60 mg |
| Total | 200 mg |

In accordance with an ordinary method, tablet preparation, containing the above-mentioned ingredients per 1 tablet was prepared.

Sample Preparation 5

| | |
|---|---|
| Type F crystals of aripiprazole anhydride | 5 mg |
| Starch | 131 mg |
| Magnesium stearate | 4 mg |
| Lactose | 60 mg |
| Total | 200 mg |

In accordance with an ordinary method, tablet preparation, containing the above-mentioned ingredients per 1 tablet was prepared.

Sample Preparation 6

| | |
|---|---|
| Type G crystals of aripiprazole anhydride | 5 mg |
| Starch | 131 mg |
| Magnesium stearate | 4 mg |
| Lactose | 60 mg |
| Total | 200 mg |

In accordance with an ordinary method, tablet preparation, containing the above-mentioned ingredients per 1 tablet was prepared.

Formulation Example

The following examples used aripiprazole drug substance made by first milling or pulverizing the conventional hydrate of aripiprazole and then heating it to form the anhydrous form (anhydride B).

Formulation Example 1

Flash-melt tablets were prepared as follows:

Intragranulation:

| Ingredient | Percent w/w | Mg. per tablet |
|---|---|---|
| Xylitol (300) Xylisorb | 26 | 52 |
| Avicel® PH 102 | 12 | 24 |
| Calcium Silicate | 43.35 | 86.7 |
| Crospovidone | 3 | 6 |
| Amorphous silica | 2 | 4 |
| Aspartame | 2 | 4 |
| Wild cherry flavor | 0.15 | 0.3 |
| Tartaric acid | 2 | 4 |
| Acesulfame K | 2 | 4 |
| Magnesium stearate | 0.25 | 0.5 |
| Total weight | 92.75 | 185.5 |

The ingredients except for the magnesium stearate were blended in a commercial V-blender in geometric proportions for 5 minutes each until all were added. The magnesium stearate was then added and the mixture blended for an additional three minutes. The blended formulation was compacted at a pressure of 30-35 kgF/cm$^2$ in a commercial compactor equipped with an orifice such that the compacts therefrom are in the form of ribbons. The ribbons were passed through a 30 mesh (600 microns) screen to form stable granules of about 150 to 400 microns.

Extragranulation Ingredients :

| Ingredient | Percent w/w | Mg. per tablet |
|---|---|---|
| Intragranulation | 92.75 | 185.5 |
| Avicel® PH 200 | 3 | 6 |
| Crospovidone | 4 | 8 |
| Magnesium stearate | 0.25 | 0.5 |
| Total weight | 100 | 200 |

The intragranulation was placed in the blender and the Avicel® PH 200 and crospovidone added thereto and blended for five minutes. The magnesium stearate was then added and the mixture blended for an additional three minutes to form the final blend. Tablets compressed therefrom had a breaking force of 2.3 kP (3.5 SCU) and disintegrated in 10 seconds in 5 ml of water. The final blend formulation demonstrated excellent flow and was free of other problems such as chipping, capping and sticking. It has been found that utilizing Avicel® PH 102 for the intragranulation and Avicel® PH 200 for the extragranulation ingredient enhanced the quality of the resultant tablets.

Formulation Example 2

Flash-melt tablets containing a combination of two grades of calcium silicate were prepared as follows:

Intragranulation:

| Ingredient | Percent w/w | Mg. per tablet |
|---|---|---|
| Xylitol (300) Xylisorb | 26 | 52 |
| Avicel® PH 102 | 12 | 24 |
| Calcium Silicate (crystalline, alpha triclinic) | 33.35 | 66.7 |
| Hubersorb 600 NF (amorphous calcium silicate) | 10 | 20 |
| Crospovidone | 3 | 6 |
| Amorphous silica | 2 | 4 |
| Aspartame | 2 | 4 |
| Wild cherry flavor | 0.15 | 0.3 |
| Tartaric acid | 2 | 4 |
| Acesulfame K | 2 | 4 |
| Magnesium stearate | 0.25 | 0.5 |
| Total weight | 92.75 | 185.5 |

The ingredients except for the magnesium stearate were blended in a commercial V-blender in geometric proportions for 5 minutes each until all were added. The magnesium stearate was added and the mixture blended for an additional three minutes. The blended formulation was compacted, and screened to form stable granules in accordance with the procedure of Formulation Example 1.

Extragranulation Ingredients:

| Ingredient | Percent w/w | Mg. per tablet |
|---|---|---|
| Intragranulation | 92.75 | 185.5 |
| Avicel® PH 200 | 3 | 6 |
| Crospovidone | 4 | 8 |
| Magnesium stearate | 0.25 | 0.5 |
| Total weight | 100 | 200 |

The intragranulation was placed in the blender and the Avicel® PH 200 and crospovidone added thereto and blended for five minutes. The magnesium stearate was then added and the mixture blended for an additional three minutes to form the final blend. Tablets compressed therefrom had a breaking force of 2.0 kP (3.1 SCU) and disintegrated in 10 seconds in 5 ml of water.

Formulation Example 3

Flash-melt tablets containing aripiprazole, an antischizophrenic drug, were prepared as follows:

Intragranulation

| Ingredient | Percent w/w | Mg. per tablet |
|---|---|---|
| Aripiprazole | 15 | 30 |
| Xylitol (300) Xylisorb | 25 | 50 |
| Avicel® PH 102 | 6 | 12 |
| Calcium Silicate | 37 | 74 |
| Crospovidone | 3 | 6 |
| Amorphous silica | 2 | 4 |

(continued)

| Ingredient | Percent w/w | Mg. per tablet |
|---|---|---|
| Aspartame | 2 | 4 |
| Wild cherry flavor | 0.15 | 0.3 |
| Tartaric acid | 2 | 4 |
| Acesulfame K | 2 | 4 |
| Magnesium stearate | 0.25 | 0.5 |
| Total weight | 94.4 | 188.8 |

The ingredients except for the magnesium stearate were blended in a commercial V-blender in geometric proportions for 5 minutes each until all were added. The magnesium stearate was added and the mixture blended for an additional three minutes. The blended formulation was compacted, and screened to form stable granules in accordance with the procedure of Formulation Example 1.

Extragranulation Ingredients:

| Ingredient | Percent w/w | Mg. per tablet |
|---|---|---|
| Intragranulation | 94.4 | 188.8 |
| Avicel® PH 200 | 1.1 | 2.2 |
| Crospovidone | 4 | 8 |
| Magnesium stearate | 0.5 | 1 |
| Total weight | 100 | 200 |

The intragranulation was placed in the blender and the Avicel® PH 200 and crospovidone added thereto and blended for five minutes. The magnesium stearate was then added and the mixture blended for an additional three minutes to form the final blend. Tablets compressed therefrom had a breaking force of 2.0 kP (3.1 SCU) and disintegrated in 10 seconds in 5 ml of water.

Formulation Example 4

Flash-melt tablets containing aripiprazole were prepared as follows:

Intragranulation:

| Ingredient | Percent w/w | Mg. per tablet |
|---|---|---|
| Aripiprazole | 0.5 | 1 |
| Xylitol (300) Xylisorb | 27 | 54 |
| Avicel® PH 102 | 12 | 24 |
| Calcium Silicate | 42 | 84 |
| Crospovidone | 3 | 6 |
| Amorphous silica | 2 | 4 |
| Aspartame | 2 | 4 |
| Wild cherry flavor | 0.15 | 0.3 |
| Tartaric acid | 2 | 4 |
| Acesulfame K | 2 | 4 |
| Magnesium stearate | 0.25 | 0.5 |
| Total weight | 92.9 | 185.8 |

The ingredients except for the magnesium stearate were blended in a commercial V-blender in geometric proportions for 5 minutes each until all were added. The magnesium stearate was added and the mixture blended for an additional three minutes. The blended formulation was compacted, and screened to form stable granules in accordance with the procedure of Formulation Example 1.

Extragranulation Ingredients:

| Ingredient | Percent w/w | Mg. per tablet |
|---|---|---|
| Intragranulation | 92.9 | 185.8 |
| Avicel® PH 200 | 2.6 | 5.2 |
| Crospovidone | 4 | 8 |
| Magnesium stearate | 0.5 | 1 |
| Total weight | 100 | 200 |

The intragranulation was placed in the blender and the Avicel® PH 200 and crospovidone added thereto and blended for five minutes. The magnesium stearate was then added and the mixture blended for an additional three minutes to form the final blend. Tablets compressed therefrom had a breaking force of 2.3 kP (3.5 SCU) and disintegrated in 10 seconds in 5 ml of water.

**Claims**

1. A pharmaceutical solid oral aripiprazole preparation having

   a) at least one dissolution rate measured after storage in the open at 25°C/60% RH for six months selected from the group consisting of 70 % or more at pH 4.5 after 60 minutes, and 55 % or more at pH 5.0 after 60 minutes; or
   b) at least one dissolution rate measured after storage in the open at 40°C/75% RH for 1 week selected from the group consisting of 70 % or more at pH 4.5 after 60 minutes, and 55 % or more at pH 5.0 after 60 minutes.

2. A process for preparing a pharmaceutical solid oral preparation as defined in claim 1, which is **characterized by** wet granulating conventional Anhydrous Aripiprazole Crystals, drying the obtained granules at 70 to 100°C and sizing them, then drying the sized granules at 70 to 100°C again.

3. A process for preparing a pharmaceutical solid oral preparation as defined in claim 1, which is **characterized by** wet granulating conventional Anhydrous Aripiprazole Crystals, drying the obtained granules at 70 to 100°C and sizing them and shaping them in the form of a pharmaceutical solid oral preparation, and then drying the pharmaceutical solid oral preparation at 70 to 100°C.

4. A process for preparing a pharmaceutical solid oral preparation as defined in claim 1, which is **characterized by** wet granulating conventional Aripiprazole Hydrate Crystals, drying the obtained granules at 70 to 100°C and sizing them, then drying the sized granules at 70 to 100°C again.

5. A process for preparing a pharmaceutical solid oral preparation as defined in claim 1, which is **characterized by** wet granulating conventional Aripiprazole Hydrate Crystals, drying the obtained granules at 70 to 100°C and sizing them and shaping them in the form of a pharmaceutical solid oral preparation, and then drying the pharmaceutical solid oral preparation at 70 to 100°C.

## FIG.1

EP 3 081 216 A1

# FIG.2

EP 3 081 216 A1

# FIG.3

EP 3 081 216 A1

# FIG.4

EP 3 081 216 A1

δ (ppm)

# FIG.5

# FIG.6

EP 3 081 216 A1

# FIG.7

EP 3 081 216 A1

# FIG.8

# FIG.9

EP 3 081 216 A1

δ (ppm)

# FIG.10

FIG.11

EP 3 081 216 A1

# FIG.12

EP 3 081 216 A1

# FIG.13

EP 3 081 216 A1

DTG %/min

DTA (µV)

TERMOGRAVIMETRY (%)

149.9 °C

136.8 °C

141.8 °C

HEATING TEMPERATURE (°C)

# FIG.14

δ (ppm)

EP 3 081 216 A1

## FIG.15

# FIG.16

EP 3 081 216 A1

# FIG.17

# FIG.18

EP 3 081 216 A1

# FIG.19

δ (ppm)

EP 3 081 216 A1

# FIG.20

FIG.21

EP 3 081 216 A1

# FIG.22

149.8°C

137.5°C

TERMOGRAVIMETRY (%)

TIME (min.)

DTA (μV)

TEMPERATURE (°C)

EP 3 081 216 A1

# FIG.23

δ (ppm)

FIG.24

# FIG.25

# FIG.26

EP 3 081 216 A1

FIG.27

δ (ppm)

# FIG.28

EP 3 081 216 A1

# FIG.29

FIG.30

EP 3 081 216 A1

# FIG.31

INTENSITY [cps]

2 θ [°]

Europäisches
Patentamt

European
Patent Office

Office européen
des brevets

# EUROPEAN SEARCH REPORT

Application Number

EP 16 16 9141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 367 141 A2 (OTSUKA PHARMA CO LTD [JP]) 9 May 1990 (1990-05-09) * page 16; claim 22 * | 1-5 | INV. A61K31/496 C07D215/22 A61P25/18 |
| X,P | EP 1 145 711 A1 (SQUIBB BRISTOL MYERS CO [US]) 17 October 2001 (2001-10-17) * claim 21; examples 3, 4 * | 1-5 | |
| E | WO 03/030868 A1 (SQUIBB BRISTOL MYERS CO [US]) 17 April 2003 (2003-04-17) * claims 36-45 * | 1-5 | |

TECHNICAL FIELDS
SEARCHED      (IPC)

C07D
A61K
A61P

The present search report has been drawn up for all claims

| Place of search | Date of completion of the search | Examiner |
|---|---|---|
| Munich | 29 June 2016 | Gregoire, Ariane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

....................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 16 9141

This annex lists the patent family members relating to the patent documents cited in the above-mentioned European search report.
The members are as contained in the European Patent Office EDP file on
The European Patent Office is in no way liable for these particulars which are merely given for the purpose of information.

29-06-2016

| Patent document cited in search report | | | Publication date | Patent family member(s) | | | Publication date |
|---|---|---|---|---|---|---|---|
| EP 0367141 | A2 | | 09-05-1990 | CN | 1042537 | A | 30-05-1990 |
| | | | | DE | 68925405 | D1 | 22-02-1996 |
| | | | | DE | 68925405 | T2 | 27-06-1996 |
| | | | | DE | 122004000043 | I1 | 03-02-2005 |
| | | | | DK | 539789 | A | 01-05-1990 |
| | | | | EP | 0367141 | A2 | 09-05-1990 |
| | | | | ES | 2084594 | T3 | 16-05-1996 |
| | | | | HK | 1002706 | A1 | 11-09-1998 |
| | | | | NL | 300161 | I1 | 01-12-2004 |
| | | | | US | 5006528 | A | 09-04-1991 |
| EP 1145711 | A1 | | 17-10-2001 | AR | 024384 | A1 | 02-10-2002 |
| | | | | AT | 328584 | T | 15-06-2006 |
| | | | | AU | 752214 | B2 | 12-09-2002 |
| | | | | AU | 4373300 | A | 18-10-2001 |
| | | | | BG | 65007 | B1 | 29-12-2006 |
| | | | | BR | 0003158 | A | 18-12-2001 |
| | | | | CA | 2311734 | A1 | 12-10-2001 |
| | | | | CN | 1317309 | A | 17-10-2001 |
| | | | | CO | 5190672 | A1 | 29-08-2002 |
| | | | | CZ | 20002391 | A3 | 14-11-2001 |
| | | | | DE | 60028536 | T2 | 16-05-2007 |
| | | | | DK | 1145711 | T3 | 02-10-2006 |
| | | | | EE | 200000497 | A | 17-12-2001 |
| | | | | EG | 23943 | A | 22-01-2008 |
| | | | | EP | 1145711 | A1 | 17-10-2001 |
| | | | | EP | 1566174 | A2 | 24-08-2005 |
| | | | | ES | 2265836 | T3 | 01-03-2007 |
| | | | | GE | P20022851 | B | 10-07-2002 |
| | | | | HK | 1039572 | A1 | 12-01-2007 |
| | | | | HU | 0002316 | A2 | 28-11-2002 |
| | | | | ID | 29837 | A | 18-10-2001 |
| | | | | IL | 136901 | A | 20-03-2008 |
| | | | | IN | 188856 | B | 16-11-2002 |
| | | | | JP | 3773763 | B2 | 10-05-2006 |
| | | | | JP | 2001302499 | A | 31-10-2001 |
| | | | | KR | 20010096450 | A | 07-11-2001 |
| | | | | LT | 2000064 | A | 25-10-2001 |
| | | | | LV | 12731 | A | 20-10-2001 |
| | | | | MY | 125766 | A | 30-08-2006 |
| | | | | NO | 20003196 | A | 15-10-2001 |
| | | | | NZ | 505302 | A | 21-12-2001 |
| | | | | PE | 02982001 | A1 | 20-05-2001 |
| | | | | PL | 341466 | A1 | 22-10-2001 |
| | | | | PT | 1145711 | E | 30-11-2006 |
| | | | | RO | 118563 | B1 | 30-07-2003 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 16 16 9141

This annex lists the patent family members relating to the patent documents cited in the above-mentioned European search report.
The members are as contained in the European Patent Office EDP file on
The European Patent Office is in no way liable for these particulars which are merely given for the purpose of information.

29-06-2016

| Patent document cited in search report | Publication date | Patent family member(s) | | Publication date |
|---|---|---|---|---|
| | | RU | 2201216 C2 | 27-03-2003 |
| | | SG | 108230 A1 | 28-01-2005 |
| | | SG | 117445 A1 | 29-12-2005 |
| | | SI | 20511 A | 31-10-2001 |
| | | SK | 9732000 A3 | 06-11-2001 |
| | | TR | 200001810 A2 | 21-11-2001 |
| | | TW | I262799 B | 01-10-2006 |
| | | UA | 63993 C2 | 15-10-2001 |
| | | US | 2010016448 A1 | 21-01-2010 |
| | | US | 2011217342 A1 | 08-09-2011 |
| | | US | 2013296337 A1 | 07-11-2013 |
| | | UY | 26306 A1 | 28-12-2001 |
| | | ZA | 200003121 A | 21-12-2001 |
| WO 03030868 A1 | 17-04-2003 | US | 2002076437 A1 | 20-06-2002 |
| | | WO | 03030868 A1 | 17-04-2003 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4734416 A
- US 5006528 A

- JP 2191256 A
- JP 2001348276 A

**Non-patent literature cited in the description**

- *Proceedings of the 4th Japanese-Korean Symposium on Separation Technology,* 06 October 1996